(19) Europäisches Patentamt
European Patent Office
Office européen des brevets

(11) EP 1 749 537 B1

(12) **FASCICULE DE BREVET EUROPEEN**

(45) Date de publication et mention de la délivrance du brevet:
**09.11.2011 Bulletin 2011/45**

(21) Numéro de dépôt: **05292669.8**

(22) Date de dépôt: **14.12.2005**

(51) Int Cl.:
***A61K 39/395*** *(2006.01)* ***A61P 7/04*** *(2006.01)*
***C07K 16/42*** *(2006.01)* ***G01N 33/53*** *(2006.01)*
***C12N 5/10*** *(2006.01)*

(54) **Anticorps anti-idiotypique neutralisant l'activité inhibitrice d'un anticorps inhibiteur du facteur VIII**

Antiidiotypische Antikörper mit neutralisierender Wirkung gegen Faktor VIII-inhibierende Antikörper

Anti-idiotypics antibodies having a neutralising activity against factor VIII inhibitor antibody

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **04.08.2005 FR 0508320**

(43) Date de publication de la demande:
**07.02.2007 Bulletin 2007/06**

(83) **Déclaration conformément à la règle 32(1) CBE (solution de l'expert)**

(73) Titulaire: **LFB Biotechnologies**
**91940 Les Ulis (FR)**

(72) Inventeurs:
- **Behrens, Christian**
  **91120 Palaiseau (FR)**
- **Gilles, Jean Guy G.**
  **1090 Bruxelles (BE)**
- **Jacquemin, Marc G.**
  **5330 Sart-Bernard (BE)**
- **Saint-Remy, Jean-Marie R.**
  **1390 Grez-Doiceau (BE)**

(74) Mandataire: **Hirsch & Associés**
**58, avenue Marceau**
**75008 Paris (FR)**

(56) Documents cités:
- **LUBAHN B C ET AL: "Characterization of a monoclonal anti-idiotype antibody to human anti-factor VIII antibodies" PROCEEDINGS OF THE NATIONAL ACADEMY OF SCIENCES OF USA, NATIONAL ACADEMY OF SCIENCE, WASHINGTON, DC, US, vol. 87, no. 21, novembre 1990 (1990-11), pages 8232-8236, XP002227743 ISSN: 0027-8424**
- **J.F. HEALEY AND AL: "Residues 484-508 contain a major determinant of the inhibitory epitope in the A2 domain of human factor VIII" THE JOURNAL OF BIOLOGICAL CHEMISTRY, vol. 270, no. 24, 1995, pages 14505-14509, XP002371336**
- **LUBAHN B C ET AL: "Characterization of a monoclonal anti-idiotype antibody to human anti-factor VIII antibodies", PROCEEDINGS OF THE NATIONAL ACADEMY OF SCIENCES OF USA. (PNAS), NATIONAL ACADEMY OF SCIENCE, WASHINGTON, DC, US LNKD- DOI: 10.1073/PNAS.87.21.8232, vol. 87, no. 21, 1 November 1990 (1990-11-01), pages 8232-8236, XP002227743, ISSN: 0027-8424**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

Art antérieur et introduction

La présente demande décrit un anticorps monoclonal anti-idiotypique dirigé contre un anticorps inhibiteur du facteur VIII se liant au domaine A2 du facteur VIII, ainsi qu'une lignée cellulaire produisant cet anticorps monoclonal anti-idiotypique, l'utilisation de cet anticorps monoclonal anti-idiotypique comme médicament, et plus particulièrement son utilisation pour la fabrication d'un médicament destiné au traitement de l'hémophilie A.

L'hémophilie A est une affection héréditaire liée à une anomalie du chromosome X, qui se traduit par une incapacité à coaguler chez les personne atteintes. Cette maladie est le résultat de mutations sur le gène d'une protéine.intervenant dans la coagulation, le facteur VIII (FVIII), qui déterminent soit une absence totale du facteur VIII dans le sang, soit un déficit partiel. L'hémophilie A est la plus commune des déficiences affectant la coagulation sanguine : elle touche en France 1 homme sur 5000, ce qui représente 80% des patients atteints d'hémophilie. L'autre type d'hémophilie, l'hémophile B, touche 20% des patients atteints d'hémophilie ; elle est causée par une déficience en un autre facteur de coagulation, le facteur IX.

Le traitement actuel de l'hémophilie (de type A ou B) consiste à administrer par voie intraveineuse le facteur de coagulation déficient ou manquant. En France, le facteur VIII destiné au traitement des hémophiles est disponible sous forme de médicaments dérivés du sang fournis par le Laboratoire Français du Fractionnement et des Biotechnologies (LFB) ou des laboratoires pharmaceutiques internationaux, ainsi que sous forme de médicaments recombinants issus du génie génétique. En effet, l'ADN codant pour le facteur VIII a été isolé et exprimé dans des cellules de mammifères (Wood et al., Nature (1984) 312 : 330-337), et sa séquence en acides aminés déduite à partir de l'ADNc.

Le facteur VIII (FVIII) sécrété est une glycoprotéine de masse moléculaire de 300 Kda (2332 acides aminés) jouant un rôle clé dans l'activation de la voie intrinsèque de la coagulation. Le FVIII inactif est constitué de six domaines : A1 (résidus 1-372), A2 (résidus 373-740), B (résidus 741-1648), A3 (résidus 1690-2019), C1 (résidus 2020-2172), et C2 (résidus 2173-2332), de l'extrémité N-terminale à l'extrémité C-terminale. Après sécrétion, le FVIII interagit avec le facteur von Willebrand (vWF) qui le protège des protéases plasmatiques. Le FVIII se dissocie du vWF après clivage par la thrombine. Ce clivage aboutit à l'élimination du domaine B et à la formation d'un hétérodimère. C'est sous cette forme que le FVIII circule dans le plasma. Cet hétérodimère est constitué d'une chaîne lourde (A1, A2) et d'une chaîne légère (A3, C1, C2).

Lorsqu'il est perfusé à un patient hémophile, le facteur VIII se fixe sur le facteur von Willebrand dans la circulation sanguine du patient.Le facteur VIII activé agit comme cofacteur du facteur IX activé, accélérant la conversion du facteur X en facteur X activé. Le facteur X activé convertit la prothrombine en thrombine. La thrombine convertit alors le fibrinogène en fibrine et un caillot apparaît.

Le problème majeur rencontré lors de l'administration de facteur VIII est l'apparition chez le patient d'anticorps dirigés contre le facteur VIII, appelés « anticorps inhibiteurs ». Ces anticorps neutralisent l'activité procoagulante du facteur VIII, qui est rendu inactif dés qu'il est perfusé. Ainsi, le facteur de coagulation administré est détruit avant d'avoir pu enrayer l'hémorragie, ce qui constitue une complication grave de l'hémophilie, le traitement devenant inefficace. De plus, certains patients non hémophiles génétiquement peuvent développer des inhibiteurs contre le facteur VIII endogène : il s'agit d'une hémophilie acquise.

Des études ont montré que la réponse immune anti-facteur VIII est de type IgG polyclonale appartenant majoritairement à la sous-classe IgG4 et IgG1 et plus rarement IgG2. Les IgG3 ne sont jamais représentées. La chaîne légère est souvent de type Kappa. La surreprésentation des IgG4 est plus accentuées chez les hémophiles qui ont un inhibiteur installé depuis longtemps. Les domaines C2 et A2 de la molécule de FVIII sont les cibles privilégiées de la réponse immune bien que dans certains cas on détecte des anticorps dirigés contre le domaine A3. En passant le plasma de patients hémophiles au travers d'une colonne d'immunoadsorption sur laquelle est immobilisé du FVIII, il est possible de purifier les anticorps totaux anti-FVIII. Les quantités récoltées dépassent souvent 100μg par 10mg d'IgG totales (Gilles JG et al. (1993) Blood ; 82 : 2452-2461). Un modèle animal a été développé pour étudier la formation d'inhibiteurs du facteur VIII ; des rats immunisés avec du facteur VIII humain recombinant montrent une réponse immune rapide, de type polyclonale (Jarvis et al. Thromb Haemost. 1996 Feb;75(2):318-25). Les mécanismes par lesquels les anticorps anti-facteur VIII interfèrent avec la fonction du facteur VIII sont nombreux, et incluent l'interférence dans le clivage protéolytique du facteur VIII et dans l'interaction du facteur VIII avec différents partenaires comme le facteur Von Willebrand (vWF), les phospholipides (PL), le facteur IX, le facteur X activé (FXa) ou l'APC (Activated Protein C).

Il existe plusieurs traitements permettant d'atténuer les conséquences de cette réponse immune, comme par exemple les traitements impliquant la desmopressine qui est une hormone synthétique stimulant la production de facteur VIII, les agents promoteurs de la coagulation comme les concentrés de complexes prothrombiques ou les concentrés de complexes prothrombiques activés, le facteur VIIa recombinant, la plasmaphérèse et les infusions de quantités importantes ou intermédiaires de facteur VIII. Toutefois, ces méthodes restent très coûteuses et peu efficaces.

Du fait de la complexité de l'analyse *in vivo* de cette réponse immune polyclonale, des équipes ont isolé des anticorps monoclonaux dirigés contre certains domaines du facteur VIII. Ainsi, un anticorps humain monoclonal de type IgG4kappa, LE2E9, a été isolé. Cet anticorps est dirigé contre le domaine C1 du facteur VIII et inhibe l'activité cofacteur du facteur VIII et sa liaison au facteur Von Willebrand (Jacquemin et al. (2000) Blood 95:156-163). De la même façon, un anticorps monoclonal humain dirigé contre le domaine C2 du facteur VIII, nommé B02C11 (IgG4kappa), produit à partir d'un répertoire de cellules B mémoire d'un patient atteint d'hémophilie A avec inhibiteurs, a été isolé (Jacquemin et al. Blood 1998 Jul 15;92(2):496-506). BO2C11 reconnaît le domaine C2 du facteur VIII et inhibe sa liaison au facteur Von Willebrand et aux phospholipides. Il inhibe complètement l'activité procoagulante du facteur VIII natif et activé. Un autre exemple d'anticorps monoclonal est l'anticorps BOIIB2, dirigé contre le domaine A2 du facteur VIII. L'anticorps BOIIB2 inhibe à 99% l'activité du facteur VIII. En se liant au domaine A2, il peut interférer et inhiber la fixation du FIXa qui possède un site de fixation de faible affinité dans cette région du FVIII et dès lors inhiber l'activité enzymatique du FIXa. Le deuxième mode d'action envisageable est son interférence dans l'équilibre entre la forme hétéro-dimère (A2:A1 et A3:C1:C2) du FVIII et la forme hétéro-trimère (A2 et A1 et A3:C1:C2) du FVIII en accélérant la dissociation du domaine A2 de ces complexes les rendant non fonctionnels. (Ananyeva NM et al (2004) Blood Coagul Fibrinolysis. Mar; 15(2) :109-24. Review).

Grâce à ces nouveaux outils, une autre stratégie de lutte contre les anticorps inhibiteurs du facteur VIII, plus récente, envisage l'administration d'anticorps anti-idiotypiques (anticorps ayant la capacité d'interagir avec la région variable d'autres anticorps) neutralisant les anticorps inhibiteurs (Saint-Rémy JM et al. (1999) Vox Sang ; 77 (suppl 1) : 21-24). Un anticorps anti-idiotypique murin, le 14C12, décrit dans le document WO 2004/014955, neutralise *in vivo* de façon dose-dépendante les propriétés inhibitrices de l'anticorps anti-facteur VIII cible (l'anticorps monoclonal BO2C11), qui est dirigé contre le domaine C2 du facteur VIII. Toutefois, la réponse immune anti-facteur VIII est polyclonale, et les anticorps inhibiteurs du facteur VIII développés par un patient ne sont pas nécessairement tous dirigés contre le domaine C2 du facteur VIII. Un traitement consistant en l'administration d'anticorps 14C12 seuls ne pourrait neutraliser que de façon partielle la réponse immune anti-facteur VIII développée chez le patient.

Une autre catégorie prépondérante d'anticorps inhibiteurs du facteur VIII rencontrés chez les patients atteints d'hémophilie A ayant développé des inhibiteurs est constituée par les anticorps inhibiteurs dirigés contre le domaine A2 du facteur VIII. Le domaine A2 est un domaine de 43 kD. Sa fonction demeure peu connue, mais il a été démontré que des anticorps inhibiteurs dirigés contre le domaine A2 du facteur VIII inhibent la fonction du facteur VIIIa par inhibition de la conversion du complexe FXase/FX à l'état de transition (Lollar et al. J Clin Invest. 1994 Jun; 93(6):2497-504, Fay et al. J Biol Chem. 1996 ; 271(11) : 6027-6032).

Ainsi, les anticorps inhibiteurs dirigés contre le domaine A2 du facteur VIII sont très importants dans la réponse immune dirigée contre le facteur VIII, cette réponse étant mixte et faisant intervenir de manière prépondérante des anticorps dirigés contre le domaine A2 et des anticorps dirigés contre le domaine C2 du facteur VIII.

Or, il n'existe pas d'outil permettant la neutralisation d'anticorps inhibiteurs dirigés contre le domaine A2 du facteur VIII.

Lubahn et al. (PNAS vol 87, no.21, page 8232-3236divulgve un anticorps capable de reconnaître le domaine A2 du FVIII, et divulgue également des anticorps antidiotypiques capables de se lier à cet anticorps anti-FVIII. Lubahn et al. suggère également l'utilisation thérapeutique de tels anticorps anti-idiotypiques. Les anticorps capables da se liés au FVIII décrits dans Lubahn et al. sont toutefois non neutralisants et ne correspondent pas à des anticorps inhibiteurs de FVIII. Les anticorps anti-idiotypiques décits dans Lubahn sont par conséquent incapables de neutraliser l'activité inhibitrice d'anticorps dirigés contre le facteur VIII.

C'est pourquoi le Demandeur a cherché, dans un premier temps, à mettre au point un nouvel outil de traitement de l'hémophilie A permettant la neutralisation d'anticorps inhibiteurs dirigés contre le domaine A2 du facteur VIII. Dans un deuxième temps, le Demandeur a cherché à mettre au point un nouvel outil permettant de neutraliser l'effet inhibiteur de la majeure partie des anticorps inhibiteurs développés par un patient atteint d'hémophilie A avec inhibiteurs.

Ainsi, le Demandeur a fabriqué un nouvel anticorps anti-idiotypique dirigé contre un anticorps inhibiteur du facteur VIII, cet anticorps inhibiteur se liant au domaine A2 du facteur VIII. De manière surprenante, le Demandeur a constaté que l'utilisation de l'anticorps anti-idiotypique selon l'invention simultanément avec un anticorps anti-idiotypique dirigé contre un inhibiteur se liant au domaine C2 du facteur VIII a un effet neutralisant de l'effet inhibiteur de la réponse immune anti-facteur VIII, et en particulier la réponse immune induite par les anticorps inhibiteurs dirigés contre le domaine A2 et par les anticorps inhibiteurs dirigés contre lé domaine C2 du facteur VIII.

Description détaillée de l'invention

La présente demande décrit un anticorps monoclonal anti-idiotypique dirigé contre un anticorps humain inhibiteur du facteur VIII, l'anticorps inhibiteur étant dirigé contre le domaine A2 du facteur VIII.

Par « anticorps inhibiteurs » ou « inhibiteurs » du facteur VIII, on entend désigner les anticorps qui inhibent l'activité procoagulante du facteur VIII, notamment en s'y fixant, et particulièrement un anticorps anti-facteur VIII dont l'épitope est situé sur le facteur VIII. De manière avantageuse, l'anticorps décrit dans présente demande possède la capacité de neutraliser au moins 50%, avantageusement au moins 60%, et de manière encore plus avantageuse au moins 70%, 80%, 90%, 99% ou 100% de l'activité inhibitrice de la coagulation des anticorps inhibiteurs dirigés contre le domaine A2 du facteur VIII, cibles des anticorps monoclonaux anti-idiotypiques décrits dans la présente demande. Cette capacité de neutraliser l'activité inhibitrice de la coagulation des anticorps inhibiteurs peut être déterminée en mesurant l'activité du facteur VIII en présence d'un anticorps inhibiteur et d'un anticorps anti-idiotypique dans un test tel que le « factor VIII chromogen test » (Jacquemin et al. (1998) Blood 92, 494-506) .

L'anticorps monoclonal anti-idiotypique décrit dans la présente demande peut être d'origine humaine ou animale. De plus, il peut être obtenu de différentes manières. Par exemple, des cellules produisant des anticorps anti-idiotypiques peuvent être obtenues à partir de lymphocytes de sang périphérique de patients présentant des anticorps inhibiteurs anti-facteur VIII ou à partir d'individus sains. Ces cellules peuvent être immortalisées par des techniques bien connues de l'homme du métier et sélectionnées par rapport à la capacité des anticorps anti-idiotypiques produits de neutraliser les anticorps inhibiteurs dirigés contre le facteur VIII. Un autre moyen de produire l'anticorps anti-idiotypique monoclonal décrit dans la présente demande est l'immunisation d'animaux, avantageusement des souris, par injection d'anticorps inhibiteurs du facteur VIII dirigés contre le domaine A2 du facteur VIII, puis la fusion des lymphocytes de rate avec une lignée cellulaire de myélome, avantageusement de myélome de souris, suivie par l'identification et le clonage de cultures cellulaires produisant les anticorps anti-idiotypiques dirigés contre les anticorps inhibiteurs du facteur VIII.

L'anticorps anti-idiotypique décrit dans la présente demande est dirigé contre des anticorps inhibiteurs dont le domaine variable de la chaîne lourde s'apparente à la lignée germinale DP-71 De tels anticorps inhibiteurs peuvent être obtenus à partir d'êtres humains (par exemple du sérum de patients présentant des anticorps inhibiteurs) ou d'autres espèces animales comme la souris, le cheval, la chèvre, des primates non humains, cette liste n'étant pas limitative, par immunisation avec le facteur VIII ou des fragments dérivés du facteur VIII, et plus particulièrement avec un fragment comprenant tout ou partie du domaine A2.

Avantageusement, l'anticorps inhibiteur cible de l'anticorps anti-idiotypique décrit dans la présente demande reconnaît l'épitope localisé entre les acides aminés 484 à 508 du facteur VIII. Plus précisément, l'épitope reconnu est un épitope conformationnel comprenant les résidus 484 à 508 et les résidus acide glutamique 389, 390 et 391 du facteur VIII. En effet, le domaine A2 du facteur VIII comprend les acides aminés 373-740 du facteur VIII. Ce domaine est impliqué dans la reconnaissance du facteur VIII par le facteur IX, quise lie aux domaines A2 et A3 du facteur VIII, et dans l'inactivation du facteur VIII, ce domaine étant l'un des principaux épitopes reconnus par les anticorps inhibiteurs.

De manière préférentielle, l'anticorps inhibiteur cible de l'anticorps anti-idiotypique décrit dans la présente demande est l'anticorps BOIIB2, déposé à X, sous le numéro X. L'anticorps BOIIB2 est un anticorps monoclonal humain IgG4 dirigé contre le domaine A2 du facteur VIII produit au départ de lymphocytes d'un patient présentant une hémophilie A sévère avec un taux d'inhibiteurs élevé. Cet anticorps appartient à la sous classe IgG4 et est issu de la lignée germinale DP-71. L'épitope qu'il reconnaît sur le facteur VIII se situe au niveau des résidus 484 à 508. Plus précisément, l'anticorps BOIIB2 reconnaît un épitope conformationnel comprenant les résidus 484 à 508 et les résidus acide glutamique 389, 390 et 391. L'anticorps BOIIB2 inhibe à 99% l'activité du facteur VIII en interférant dans la fonction du complexe tenase soit en inhibant la fixation du FIXa et / ou du FX au complexe tenase, soit en accélérant la dissociation du domaine A2.

Dans un premier objet de l'invention, la région variable de chacune des chaînes légères de l'anticorps monoclonal anti-idiotypique de l'invention est codée par une séquence d'acide nucléique possédant au moins 70% d'identité avec la séquence d'acide nucléique SEQ ID NO : 2, et la région variable de chacune des chaînes lourdes de l'anticorps monoclonal anti-idiotypique est codée par une séquence d'acide nucléique possédant au moins 70% d'identité avec la séquence d'acide nucléique SEQ ID NO : 1. De manière particulièrement avantageuse, l'identité des séquences est d'au moins 80%, et de manière préférée d'au moins 95 à 99% d'identité. Le pourcentage d'identité est calculé en alignant 2 séquences à comparer et en comptant le nombre de positions possédant un nucléotide identique, ce nombre étant divisé par le nombre de nucléotides total de la séquence. La dégénérescence du code génétique peut être à l'origine du fait qu'un même acide aminé puisse être codé par plusieurs triplets de nucléotides différents. En tout état de cause, ces différences de séquences n'affectent en rien l'affinité de l'anticorps monoclonal pour sa cible, ni sa capacité à neutraliser l'activité inhibitrice des anticorps inhibiteurs cibles.

Dans un aspect préféré de l'invention, la région variable de chacune des chaînes légères de l'anticorps monoclonal anti-idiotypique est codée par la séquence d'acide nucléique SEQ ID NO : 2, et la région variable de chacune des chaînes lourdes l'anticorps monoclonal anti-idiotypique est codée par la séquence d'acide nucléique SEQ ID NO : 1.

De manière avantageuse, la séquence peptidique de chacune des régions variables des chaînes légères de l'anticorps selon l'invention est une séquence possédant au moins 70% d'identité, et de manière avantageuse au moins 80% ou 90%, et de manière encore plus avantageuse au moins 99% d'identité avec la séquence SEQ ID NO : 4.

De manière avantageuse, la séquence peptidique de chacune des régions variables des chaînes lourdes de l'anticorps selon l'invention est une séquence possédant au moins 70% d'identité, et de manière avantageuse au moins 80% ou 90%, et de manière encore plus avantageuse au moins 99% d'identité avec la séquence SEQ ID NO : 3.

De manière particulièrement avantageuse, la séquence peptidique de chacune des chaînes légères de l'anticorps selon l'invention est une séquence possédant au moins 70% d'identité, et de manière avantageuse au moins 80% ou 90%, et de manière encore plus avantageuse au moins 99% d'identité avec la séquence SEQ ID NO : 4, et la séquence peptidique de chacune des chaînes lourdes de l'anticorps selon l'invention est une séquence possédant au moins 70% d'identité, et de manière avantageuse au moins 80% ou 90%, et de manière encore plus avantageuse au moins 99% d'identité avec la séquence SEQ ID NO : 3.

De manière préférée, la séquence peptidique de chacune des chaînes légères de l'anticorps selon l'invention est la séquence SEQ ID NO : 4.

De manière préférée, la séquence peptidique de chacune des chaînes légères de l'anticorps selon l'invention est la séquence SEQ ID NO : 3.

La séquence peptidique déduite de la séquence SEQ ID NO : 2 est la séquence SEQ ID NO : 4 et la séquence peptidique déduite de la séquence SEQ ID NO : 1 est la séquence SEQ ID NO : 3. De manière préférentielle, la région variable de chacune des chaînes légères de l'anticorps monoclonal anti-idiotypique selon l'invention possède la séquence peptidique SEQ ID NO : 4 et la région variable de chacune des chaînes lourdes de l'anticorps monoclonal anti-idiotypique selon l'invention possède la séquence peptidique SEQ ID NO : 3.

L'anticorps selon l'invention s'entend aussi de tout anticorps modifié répondant aux caractéristiques de l'invention, dans lequel un ou plusieurs acides aminés ont été substitué(s) ou délété(s). Une telle substitution ou délétion peut être localisée à n'importe quelle position dans la molécule. Dans le cas où plusieurs acides aminés ont été substitués ou délétés, toute combinaison de substitution ou de délétion peut être considérée. De telles altérations de la séquence des régions variables de l'anticorps selon l'invention peuvent être effectuées dans le but d'augmenter le nombre de résidus susceptibles d'entrer en contact entre l'anticorps anti-idiotypique selon l'invention et l'anticorps inhibiteur cible.

Dans un mode de réalisation de l'invention, l'anticorps anti-idiotypique est un anticorps murin.

Avantageusement, cet anticorps monoclonal anti-idiotypique murin est une IgGlkappa.

De manière préférée, l'anticorps monoclonal selon l'invention est un anticorps chimérique. On entend par « anticorps chimérique » un anticorps dont les régions variables des chaînes légères et des chaînes lourdes appartiennent à une espèce différente des régions constantes des chaînes légères et des chaînes lourdes. Ainsi, l'anticorps selon l'invention possède en outre des régions constantes de ses chaînes légères et lourdes appartenant à une espèce non-murine. A cet égard, toutes les familles et espèces de mammifères non-murins sont susceptibles d'être utilisées, et en particulier l'homme, le singe, les muridés (sauf la souris), les suidés, les bovidés, les équidés, les félidés, les canidés, par exemple, ainsi que les oiseaux.

Les anticorps chimériques selon l'invention peuvent être construits en utilisant les techniques standard de l'ADN recombinant, bien connues de l'homme du métier, et plus particulièrement en utilisant les techniques de construction des anticorps « chimériques » décrites par exemple dans Morrison et al., Proc. Natl. Acad. Sci. U.S.A., 81, pp. 6851-55 (1984), où la technologie de l'ADN recombinant est utilisée pour remplacer la région constante d'une chaîne lourde et/ou la région constante d'une chaîne légère d'un anticorps provenant d'un mammifère non-humain avec les régions correspondantes d'une immunoglobuline humaine.

Dans un aspect particulier de l'invention, l'anticorps selon l'invention est un anticorps hybride humain, c'est-à-dire un anticorps chimérique dont la partie constante est humaine. Ce mode de réalisation de l'invention permet de diminuer l'immunogénicité de l'anticorps chez l'homme et par là même d'améliorer son efficacité lors de son administration thérapeutique chez l'homme. Avantageusement, l'anticorps selon l'invention est un anticorps humanisé. Un tel anticorps peut être obtenu par association d'une ou plusieurs région(s) CDR (complementarity determining région) d'un anticorps monoclonal d'une espèce non-humaine avec des régions framework (régions très conservées des régions variables, nommées aussi "charpente") humaines, un tel procédé d'obtention étant bien enseigné dans l'état de la technique (Jones et al., Nature (1986) 321:522 ; Riechmann et al., Nature (1988) 332:323).

De manière avantageuse, l'anticorps monoclonal anti-idiotypique selon l'invention est l'anticorps 30D1 produit par l'hybridome 30D1 déposé sous le numéro d'enregistrement CNCM I-3450 à la Collection Nationale de Cultures de Microorganismes (CNCM). La région variable de chacune des chaînes légères l'anticorps monoclonal anti-idiotypique 30D1 est codée par la séquence d'acide nucléique SEQ ID NO : 2, et la région variable de chacune des chaînes lourdes l'anticorps monoclonal anti-idiotypique 30D1 est codée par la séquence d'acide nucléique SEQ ID NO : 1. La méthode d'obtention de l'hybridome 30D1 est décrite dans la partie « Exemples » du présent document.

L'anticorps monoclonal anti-idiotypique selon l'invention s'entend aussi de tout anticorps comprenant des fragments de l'anticorps 30D1, et plus particulièrement tout anticorps comprenant la région variable de la chaîne légère et/ou la région variable de la chaîne lourde de l'anticorps 30D1, ou tout fragment de la région variable de la chaîne légère et/ou la région variable de la chaîne lourde de l'anticorps 30D1. Par « fragments » on entend désigner un fragment F(ab')2 ou un fragment Fab' ou un fragment Fab ou une région CDR ( complementarity determining region) ou toute version modifiée de l'un quelconque de ces fragments ou région.

Dans un mode de réalisation particulier de l'invention, l'anticorps monoclonal anti-idiotypique selon l'invention est un fragment F(ab')2 ou un fragment Fab' ou un fragment Fab ou une région CDR (complementarity determining region) ou toute version modifiée de l'un quelconque de ces fragments ou région. La digestion enzymatique des immunoglobulines par la papaine génère 2 fragments identiques, qu'on appelle « fragment Fab » (Fragment Antigen Binding), et un fragment Fc (fraction cristallisable). Le fragment Fc est le support des fonctions effectrices des immunoglobulines.

Par digestion à la pepsine, un fragment F(ab')2 est généré, où les deux fragments Fab restent liés par deux ponts disulfure, et le fragment Fc est scindé en plusieurs peptides. Le fragment F(ab')2 est formé de deux fragments Fab' (un fragment Fab' consistant en un Fab et une région charnière), liés par des ponts disulfure intercaténaires pour former un F(ab')2.

De tels fragments, qui contiennent le site de liaison de l'anticorps, peuvent avoir perdu un certain nombre de propriétés de l'anticorps entier duquel ils sont issus, comme la capacité d'activer le complément ou de lier les récepteurs Fcgamma. Toutefois, ces fragments n'ont pas perdu la capacité de l'anticorps entier de neutraliser l'anticorps inhibiteur. Ainsi, l'invention s'entend aussi des fragments F(ab')2, Fab', Fab, de la région CDR ou toute version modifiée de l'un quelconque de ces fragments ou région, de l'anticorps 30D1. En particulier, ces fragments ont conservé la capacité de l'anticorps entier de neutraliser les anticorps BOIIB2.

Un autre objet de l'invention est une lignée cellulaire stable produisant un anticorps tel que décrit précédemment. La lignée cellulaire stable selon l'invention peut être d'origine humaine ou animale. La lignée cellulaire stable selon l'invention peut provenir de cellules humaines immortalisées. Dans un autre mode de réalisation de l'invention, cette lignée peut provenir de cellule d'origine animale, par exemple de souris, immortalisées. Un exemple préféré d'une lignée issue de ce mode de réalisation de l'invention est la lignée 30D1, déposée à la CNCM sous le numéro I-3450. Dans un autre mode de réalisation, la lignée cellulaire stable selon l'invention est une lignée qui a intégré une construction génétique permettant l'expression de l'anticorps selon l'invention à l'endroit désiré du génome. L'étape qui consiste à obtenir une telle cellule est une transfection stable. Cette étape peut être appliquée sur n'importe quel type de cellules pourvu qu'elles puissent être maintenues en culture in vitro. La transfection stable nécessite l'intégration de la construction génétique, qui peut être effectuée par recombinaison homologue ou peut se faire de manière aléatoire. C'est la présence d'une cassette de sélection positive dans la construction génétique comportant le gène d'intérêt qui confère à la cellule la résistance à un antibiotique par exemple qui atteste de l'insertion du transgène dans le génome cellulaire. A l'issue d'une étape de sous-clonage, on obtient une lignée cellulaire productrice à long terme de l'anticorps de l'invention, par exemple 30D1, pouvant être maintenue en culture in vitro.

La lignée cellulaire stable exprimant un anticorps selon l'invention peut être choisie parmi le groupe consistant en une lignée cellulaire humaine, une lignée de rongeur, par exemple une lignée murine, SP2/0, YB2/0, IR983F, un myélome humain comme Namalwa ou toute autre cellule d'origine humaine comme PERC6, les lignées CHO, notamment CHO-K-1, CHO-Lec10, CHO-Lec1, CHO-Lec13, CHO Pro-5, CHO dhfr- (CHO DX B11, CHO DG44), ou d'autres lignées choisies parmi Wil-2, Jurkat, Vero, Molt-4, COS-7, 293-HEK, BHK, K6H6, NS0, SP2/0-Ag 14 et P3X63Ag8.653.

Un autre objet particulier de l'invention est l'hybridome 30D1 déposé sous le numéro d'enregistrement CNCM I-3450 à la Collection Nationale de Cultures de Microorganismes (CNCM). La région variable de chacune des chaînes légères de l'anticorps monoclonal anti-idiotypique produit par l'hybridome 30D1 est codée par la séquence d'acide nucléique SEQ ID NO : 2, et la région variable de chacune des chaînes lourdes de l'anticorps monoclonal anti-idiotypique produit par l'hybridome 30D1 est codée par la séquence d'acide nucléique SEQ ID NO : 1. L'anticorps produit par l'hybridome 30D1 est l'anticorps 30D1, et une méthode d'obtention de l'hybridome 30D1 est décrite dans la partie « Exemples » du présent document. Un autre objet de l'invention est un fragment d'ADN de séquence SEQ ID NO : 1 codant pour la région variable de la chaîne lourde de l'anticorps selon l'invention tel que décrit précédemment. Ce fragment d'ADN peut être inséré dans un vecteur permettant l'expression d'un polypeptide, préférentiellement d'un anticorps, dont la région variable de la chaîne lourde est codée par la séquence d'acide nucléique SED ID NO : 1, dont la séquence peptidique déduite est la séquence SEQ ID NO : 3, afin de l'introduire et de le maintenir dans une cellule hôte. Ce vecteur permet l'expression de ce fragment d'acide nucléique étranger dans la cellule hôte car il possède des séquences indispensables (promoteur, séquence de polyadénylation, gène de sélection) à cette expression.

De tels vecteurs sont bien connus de l'homme du métier, et peuvent être un adénovirus, un rétrovirus, un plasmide ou un bactériophage, cette liste n'étant pas limitative. De plus, toute cellule de mammifère peut être utilisée comme cellule hôte, c'est-à-dire comme cellule exprimant le polypeptide ou l'anticorps selon l'invention, par exemple SP2/0, YB2/0, IR983F, un myélome humain comme Namalwa ou toute autre cellule d'origine humaine comme PERC6, les lignées CHO, notamment CHO-K-1, CHO-Lec10, CHO-Lec1, CHO-Lec13, CHO Pro-5, CHO dhfr- (CHO DX B11, CHO DG44), ou d'autres lignées choisies parmi Wil-2, Jurkat, Vero, Molt-4, COS-7, 293-HEK, BHK, K6H6, NS0, SP2/0-Ag 14 et P3X63Ag8.653.

Un autre objet de l'invention est un fragment d'ADN de séquence SEQ ID NO : 2 codant pour la région variable de la chaîne légère d'un anticorps selon l'invention tel que décrit précédemment. Ce fragment d'ADN peut être inséré dans un vecteur permettant l'expression d'un polypeptide, préférentiellement d'un anticorps, dont la région variable de la chaîne légère est codée par la séquence d'acide nucléique SED ID NO : 2, dont la séquence peptidique déduite est la séquence SEQ ID NO : 4, afin de l'introduire et de le maintenir dans une cellule hôte. Ce vecteur permet l'expression de ce fragment d'acide nucléique étranger dans la cellule hôte car il possède des séquences indispensables (promoteur, séquence de polyadénylation, gène de sélection) à cette expression. De tels vecteurs sont bien connus de l'homme du métier, et peuvent être un adénovirus, un rétrovirus, un plasmide ou un bactériophage, cette liste n'étant pas limitative.

De plus, toute cellule de mammifère peut être utilisée comme cellule hôte, c'est-à-dire comme cellule exprimant le polypeptide ou l'anticorps selon l'invention, par exemple SP2/0, YB2/0, IR983F, un myélome humain comme Namalwa ou toute autre cellule d'origine humaine comme PERC6, les lignées CHO, notamment CHO-K-1, CHO-Lec10, CHO-Lec1 CHO-Lec13, CHO Pro-5, CHO dhfr- (CHO DX B11, CHO DG44), ou d'autres lignées choisies parmi Wil-2, Jurkat, Vero, Molt-4, COS-7, 293-HEK, BHK, K6H6, NS0, SP2/0-Ag 14 et P3X63Ag8.653.

Un autre objet de l'invention est une composition pharmaceutique comprenant un anticorps selon l'invention et au moins un excipient et/ou au moins un véhicule pharmaceutiquement acceptable. De manière préférentielle, l'anticorps monoclonal anti-idiotypique contenu dans la composition pharmaceutique selon l'invention est l'anticorps 30D1, un fragment ou région dérivée d'30D1, ou encore un anticorps chimérique ou humanisé comportant les régions variables d'30D1 et tel que décrit précédemment dans le présent document. La composition pharmaceutique selon l'invention peut être formulée dans tout excipient qu'un patient à traiter peut tolérer. Des exemples de tels excipients incluent l'eau, les solutions salines, la solution de Ringer, les solutions de dextrose, et toute autre solution aqueuse physiologique appropriée. L'excipient peut aussi contenir de faibles quantités d'additifs tels que des substances qui augmentent l'isotonicité et la stabilité de la composition. De tels excipients incluent le tampon phosphate, le tampon bicarbonate, et le tampon Tris. De tells excipients sont bien connus de l'homme du métier. Des formulations standard peuvent se présenter sous forme de liquides injectables ou de formulations solides qui peuvent être re-suspendues dans un liquide approprié avant administration. Les véhicules pouvant être utilisés pour la préparation de la composition pharmaceutique selon l'invention ont avantageusement pour fonction d'augmenter la demi-vie de la composition thérapeutique dans l'animal ou le patient, ou pour permettre le relargage contrôlé du principe actif. De tels véhicules peuvent être des polymères organiques et synthétiques et d'autres composés chimiques qui peuvent disséminer les médicaments à une cadence normale ou les disséminer seulement dans certains environnements, ainsi que des liposomes, cette liste n'étant pas limitative.

Avantageusement, la composition pharmaceutique selon l'invention comprend en outre au moins un anticorps anti-idiotypique dirigé contre un anticorps inhibiteur se liant à un domaine autre que le domaine A2 du facteur VIII. Cet autre anticorps peut être un anticorps anti-idiotypique dirigé contre un anticorps inhibiteur se liant au domaine A1, ou A3, ou B, C1 ou C2 du facteur VIII. En effet, un patient atteint d'hémophilie A ayant développé des anticorps inhibiteurs présente le plus souvent plusieurs types d'anticorps inhibiteurs. De plus, les quantités et la nature des différents types d'anticorps inhibiteurs ne sont pas fixes mais peuvent changer au cours de la vie du patient. Les différents anticorps inhibiteurs d'un même patient étant ainsi dirigés contre des domaines différents du facteur VIII, il est particulièrement avantageux de traiter le patient non pas avec un, mais avec plusieurs types d'anticorps anti-idiotypiques, dirigés contre des anticorps inhibiteurs différents.

De manière préférentielle, la composition pharmaceutique comporte un anticorps monoclonal anti-idiotypique dirigé contre un anticorps inhibiteur se liant au domaine C2 du facteur VIII et l'anticorps monoclonal selon l'invention. En effet, les domaines A2 et C2 représentent les cibles principales de la réaction immunitaire anti-facteur VIII. Ainsi, une composition pharmaceutique comportant un mélange d'anticorps anti-idiotypiques dirigés contre des anticorps inhibiteurs se liant au domaine A2 du facteur VIII et d'anticorps anti-idiotypiques dirigés contre des anticorps inhibiteurs se liant au domaine C2 permet la neutralisation d'au moins 70%, et avantageusement au moins 80% ou 90% de la totalité des anticorps inhibiteurs présents chez un patient. Dans un mode de réalisation préféré de l'invention, la composition pharmaceutique selon l'invention comporte l'anticorps 14C12 (déposé sous le numéro LMBP 5878CB à la Belgian Coordinated Collections of Microorganisms) et l'anticorps 30D1. Dans un autre mode de réalisation préféré de l'invention, la composition pharmaceutique comporte l'anticorps 14C12 et un anticorps chimérique ou humanisé dérivé de l'anticorps 30D1, c'est-à-dire un anticorps comportant les régions variables de l'anticorps 30D1

Un autre objet de l'invention est l'utilisation de l'anticorps selon l'invention pour son utilisation comme médicament.

Un autre objet de l'invention est l'utilisation de l'anticorps selon l'invention pour la fabrication d'un médicament. Avantageusement, un tel médicament est utilisé pour réduire et/ou prévenir et/ou traiter les saignements chez un patient atteint d'hémophilie comportant des anticorps inhibiteurs dirigés contre le domaine A2 du facteur VIII.

Un autre objet de l'invention est l'utilisation de l'anticorps selon l'invention pour la fabrication d'un médicament destiné au traitement de l'hémophilie de type A.

Avantageusement, l'hémophilie de type A ainsi traitée est une hémophilie avec inhibiteurs. Ce type d'hémophilie traitée par l'anticorps selon l'invention peut être innée ou acquise. L'anticorps selon l'invention, en neutralisant les anticorps inhibiteurs, rend au traitement par injection de facteur VIII au patient son efficacité, l'activité du facteur VIII n'étant plus inhibée par les anticorps inhibiteurs.

Un autre objet de l'invention est l'utilisation de l'anticorps selon l'invention pour neutraliser in vitro ou in vivo l'activité inhibitrice d'un anticorps inhibiteur dirigé contre le domaine A2 du facteur VIII. Ce procédé peut être mis en oeuvre pour dépléter le sang d'un patient de ses anticorps inhibiteurs dirigés contre le domaine A2 du facteur VIII, le sang étant ensuite réinjecté audit patient.

Un autre objet de l'invention est l'utilisation de l'anticorps pour adsorber les anticorps inhibiteur, à titre d'exemple afin de purifier des anticorps inhibiteurs du facteur VIII.

Enfin, un autre objet de l'invention est l'utilisation de l'anticorps selon l'invention pour la détection et/ou la purification d'anticorps inhibiteurs du facteur VIII. Les procédés de mise en oeuvre de telles méthodes de détection et de purification sont bien connues de l'homme du métier. On peut citer comme exemple l'utilisation à cet effet d'une colonne d'immuno-purification contenant des billes à la surface desquelles est greffé l'anticorps selon l'invention. Seules les molécules reconnues par l'anticorps vont se fixer sur les billes. Les autres vont traverser la colonne. Pour récupérer la molécule, il suffit d'augmenter la force ionique du solvant.

D'autres aspects et avantages de l'invention seront décrits dans les exemples qui suivent, qui doivent être considérés comme illustratifs et ne limitent pas l'étendue de l'invention.

## Description des Figures

**Figure 1** : Evaluation de la capacité de l'anticorps BOIIB2 à inhiber le facteur VIII dans un essai fonctionnel.
**Figure 2 :** épitope mapping de l'anticorps BOIIB2.
**Figure 3 :** Réponse immune des souris injectées avec l'anticorps BOIIB2 en fonction de la saignée par mesure de la densité optique.
**Figure 4** : Fixation directe des anticorps anti-idiotypiques aux anticorps BOIIB2 insolubilisés.
**Figure 5** : Inihibition de la fixation de l'anticorps BOIIB2 au facteur VIII recombinant insolubilisé.
**Figure 6** : Neutralisation de l'activité inhibitrice anti-facteur VIII de l'anticorps BOIIB2.
**Figure 7 :** Neutralisation de l'effet inhibiteur du mélange de l'anticorps BO2C11 et de l'anticorps BOIIB2 par les anticorps anti-idiotypiques 14C12 et 30D1.

## Exemples

### Exemple 1 : Production d'un anticorps monoclonal humain dirigé contre le domaine A2 du facteur VIII (« anticorps anti-A2'»)

Des anticorps humains monoclonaux possédant la spécificité et les caractéristiques désirés sont produits par transformation de lymphocytes B obtenus à partir de sang périphériques d'individus, préférentiellement de patients souffrant d'hémophilie A ou d'hémophilie acquise. Les cellules B sont transformées par infection avec le virus d'Epstein-Barr et activation d'antigènes de surface grâce à des techniques bien connues de l'homme du métier (Madec et al. (1996) J Immunol 156:3541-3549). Les surnageants cellulaires contenant les anticorps désirés sont identifiés par des tests spécifiques.

Ainsi, les anticorps dirigés contre le facteur VIII sont identifiés en faisant réagir le surnageant avec des plaques de polystyrène coatées avec du facteur VIII ou avec un complexe facteur VIII/facteur Von Willebrand. La liaison des anticorps spécifiques est détectée par l'addition d'une IgG anti-humain couplée avec une enzyme. L'addition d'un substrat enzymatique converti en un composé coloré en présence d'une enzyme permet la détection d'anticorps spécifiques. De telles méthodes, désignées par le terme ELISA (Enzyme-Linked Immuno-Sorbent Assays), sont bien connues de l'homme du métier. Une description détaillée est disponible dans l'ouvrage « Current Protocols in Immunology, Chapter 2, John Wiley & Sons, Inc, 1994 ».

Les cellules B produisant les anticorps anti-facteur VIII sont ensuite expandues et clonées par dilutions limites. Des méthodes pour le clonage sont décrites, par exemple, dans l'ouvrage « Current Protocols in Immunology, Chapter 2, John Wiley & Sons, Inc, 1994 ».

Les anticorps anti-facteur VIII possédant les caractéristiques désirées, à savoir la capacité d'inhiber l'activité procoagulante du facteur VIII, sont identifiés grâce aux kits d'essais chromogéniques commercialement disponibles, et suivant les recommandations du fabriquant. Des anticorps qui inhibent la fonctionnalité du facteur VIII et qui présentent une affinité de liaison pour le facteur VIII suffisante sont sélectionnés. La figure 1 montre la faculté d'un anticorps, nommé BOIIB2, spécifique du domaine A2 du facteur VIII, à inhiber le facteur VIII dans un essai fonctionnel. Le test fonctionnel utilisé est un test chromogénique dans lequel le facteur VIII activé par la thrombine agit comme un cofacteur au facteur IXa dans la conversion du facteur X en facteur Xa. Brièvement, 20 µl de facteur VIII recombinant (recFVIII) dilué dans une solution de PBS-BSA (1 IU/ml) est mélangé avec un volume égal d'une diultion sérielle de l'anticorps BOIIB2 dans la même solution de PBS-BSA et incubé pendant 1 heure à 37°C. 20 µl du mélange sont ensuite incubés pendant 3 minutes à température ambiante dans un puits de micortitrage avec 20 µl du réactif 1 (facteur X) et 20 µl de réactif 2 (facteur IXa) avant addition de 100 µl de réactif 3 (substrat chromogénique et tampon d'arrêt). Des expérimentations de contrôle incluant le recFVIII incubé sans anticorps spécifiques ou avec la même concentration d'anticorps non-relevants sont effectuées. La densité de coloration du substrat est directement mesurée à 405 nm avec une référence à 450 nm. Le pourcentage d'inhibition est calculé en comparaison avec le contrôle positif recFVIII 1 IU/ml. La courbe indique que le BOIIB2 inhibe le FVIII à 99% à une concentration de 0.1 µg/ml.

Alternativement, des anticorps possédant les caractéristiques requises peuvent être produits par immunisation d'animaux. Dans ce cas, le facteur VIII humain est injecté à des souris avec un adjuvant. Les anticorps monoclonaux anti-humain sont ensuite obtenus par fusion de lymphocytes de rate avec une lignée cellulaire de myélome de souris. Les surnageants cellulaires produisant les anticorps anti-facteur VIII sont identifiés et clonés par dilution limite. Une description générale de telles méthodes peut être trouvée dans « Current Protocols in Immunology, Chapter 2, John Wiley & Sons, Inc, 1994 ». D'autres sélections d'inhibiteurs possédant les caractéristiques désirées sont décrites ci-dessous.

Des anticorps produits par des souris sont humanisés. Ainsi, les séquences des parties variables murines des chaînes lourdes et légères sont alignées avec les régions variables d'immunoglobuline humaine de manière à identifier un anticorps humain possédant la meilleure homologie des régions framework (FR). Le fragment d'ADN codant pour les régions variables humanisées sont ensuite synthétisées par la méthode PCR-based CDR grafted, telle que décrite dans Sato K et al. (1993) ; Cancer Research 53: 851-856.

**Exemple 2 : Caractérisation de la spécificité et de l'affinité des anticorps anti-A2**

La spécificité des anticorps dirigés contre le domaine A2 du facteur VIII est caractérisée en utilisant un système de transcription-traduction combiné au moyen de réticulocytes de lapins. A cet égard, une banque de plasmides contenant des fragments variés du domaine A2 est construite.

La construction plasmidique pSP64-FVIII (ATCC, Rockville, MD) contenant un ADNc complet de 7,2Kb est utilisé comme matrice pour générer tous les fragments par PCR. Les fragments d'ADNc portant des mutations ou délétions sont produits par SOE-PCR (Splicing by Overlap Extension-PCR). Pour les fragments de facteur VIII de moins de 15 acides aminés, une séquence tag est ajoutée incluant l'ubiquitine et/ou l'épitope tag T7 pour la reconnaissance spécifique par des anticorps anti-tag, ainsi qu'une séquence complémentaire de cystéines pour marquage. Les fragments polypeptidiques de facteur VIII sont produits par la méthode « TNT coupled Reticulocyte Lysate Systems » (Promega), selon les instructions du fabriquant.

L'immunoprécipitation des gènes transcrits est effectuée comme suit. Des dilutions des échantillons contenant les anticorps spécifiques sont mélangés à 40 μl de Protéine A Sépharose (Pharmacia) dans 500 μl d'un tampon approprié, et le mélange est doucement agité pendant 1 heure à 4°C. Les anticorps non liés sont éliminés par une série de centrifugations et de lavages. Le complexe anticorps-Protéine A Sépharose est resuspendu dans 300 μl de tampon supplémenté par 3 μl de polypeptides de facteur VIII marqués in vitro à la méthionine L-($^{35}$S) pour une incubation à 4°C pendant 2 heures. Les complexes antigènes/anticorps sont élués des billes par ébullition pendant 3 minutes dans 30 μl de tampon dénaturant et la radioactivité comptée. Un second aliquot est analysé par SDS-PAGE et visualisé en autoradiographie.

La figure 2 montre les résultats d'une telle expérimentation pour l'anticorps BOIIB2. Les résidus portant la séquence tag des régions 379-546, 379-546Mut (R484A, Y487A, R489A, P492A) et 461-531 ont été marquées et produites par des lysats de réticulocytes. Les fragments ont été immunoprécipités par l'anticorps humain BOIIB2 lié à la Protéine-A Sépharose. Après lavage, le complexe a été élué et la radioactivité mesurée (en CPM) par scintillation et chargés sur SDS-PAGE, suivi d'une autoradiographie. La liaison de BOIIB2 au domaine A2 incluant les résidus 379-546 est montrée. En tout état de cause, la liaison a été abolie quand 4 mutations ont été introduites dans la région ou quand la région a été tronquée de sa partie N-terminale (461-531). L'anticorps anti-c-myc 9E10 et le plasma humain ont été utilisés comme contrôles positifs et négatifs respectivement.

Un site de liaison dans la région comportant les résidus 389-510 est identifié dans le domaine A2. Cependant, un tracé plus précis de l'épitope est difficile à cause du fait que l'enlèvement des parties amino-terminale ou carboxy-terminale de tels fragments abolit rapidement la liaison de l'anticorps. Nous avons donc procédé par une approche par étapes dans laquelle des mutations uniques ont été introduites dans le site de liaison présumé, à savoir 484-509. La figure 2 montre que des mutations uniques dans un tel site abolit la liaison de l'anticorps. Par ailleurs, des mutations uniques ont été introduites dans la séquence de 3 acides glutamiques situés en 389-391, qui résulte aussi dans une perte de liaison. On peut ainsi en conclure que le site de liaison est situé à l'intérieure de la séquence en acides aminés 484-509, mais que 3 résidus d'acide glutamique sont requis à une certaine distance pour une liaison efficace de l'anticorps.

L'affinité de l'anticorps est calculée en utilisant un système de « Surface Plasmon Resonance ». Ainsi, la cinétique d'interaction entre le facteur VIII et l'anticorps humain en temps réel est analysée en utilisant un instrument Pharmacia Biosensor BIAcore™ (Pharmacia Biosensore AB). L'anticorps BOIIB2 purifié (20 μg/ml dans 10mM de tampon acétate de sodium pH5.0) est immobilisé sur une surface activée d'une puce « CM5 sensor chip », selon les indications du fabricant. Toutes les expérimentations ont été effectuées en HBS à flot constant de 10 μl/min. Le facteur VIII en HBS est infusé à différentes concentrations à travers des ECR immobilisés sur une sonde de surface. A la fin de chaque cycle, la surface est régénérée par rinçage avec du HCl, pH2, pendant 36 sec. Les constantes d'association et de dissociation sont déterminées au moyen d'un logiciel d'évaluation BIA, selon des modèles supposant la liaison d'un analyte à un ou deux ligands immobilisés.

Le $k_{diss}$ de BOIIB2 a été évalué à $1.10^{-8}$ $s^{-1}$ et le $k_{ass}$ de BOIIB2 a été évalué à $1.10^{3}$ $M^{-1}$ $S^{-1}$.

**Exemple 3 séquençage de l'anticorps BOIIB2**

L'isolation de l'ARN de lignées cellulaires humaines B immortalisées par l'EBV sont produites au moyen de réactif TRIzol selon les indications du fabricant (Life Technologies). L'ADNc est synthétisé avec le système de pré-amplification SuperScript pour la synthèse du premier brin d'ADNc. L'ADNc codant la région la région variable de la chaîne lourde (VH) de l'anticorps est amplifiée par PCR au moyen d'amorces spécifiques de la séquence leader des familles VH et pour le premier exon de la région CH, comme décrit dans Jacquemin et al. (2000); Blood. Jan 1;95(1): 156-63. L'appariement est effectué à 60°C pendant 40 cycles de PCR. Les produits PCR de taille appropriée (460bp) sont isolés à partir de 1.5% de gel d'agarose et clonés au moyen du Kit TA Cloning (Invitrogen BV, Leek,

The Nederlands). Un screening PCR au moyen des couples d'amorces correspondant à la famille du gène VH d'intérêt est effectué sur des cultures de colonies randomisées. L'ADN plasmidique des colonies positives est isolé au moyen du Wizard Plus Minipreps (Promega, Menlo park, CA) et séquencé dans les 2 directions avec la Sequenase (United States Biochemical, Cleveland, OH), selon les instructions du fabriquant. L'analyse des séquences du gène de la région variable est effectuée au moyen de « V BASE Sequence Directory » (Tomlinson et al., MRC Centre for protein Engineering, Cambridge, UK).

Le gène VH de BOIIB2 appartient au groupe VH4 et est homologue de DP-71. Le segment J est homolgue de JH6c. Le séquençage du gène de la chaîne légère clonée a identifié le VL comme un VLIII et le segment J comme un JLS.

La séquence nucléotidique de la chaîne lourde du BOIIB2 est la séquence SEQ ID NO : 5, et la séquence nucléotidique de la chaîne légère de l'anticorps BOIIB2 est la séquence SEQ ID NO : 6. La séquence peptidique correspondant à la séquence SEQ ID NO : 5 est la séquence SEQ ID NO : 7 et la séquence peptidique correspondant à la séquence SEQ ID NO : 6 est la séquence SEQ ID NO : 8.

**Exemple 4 Production de l'anticorps anti-idiotypique 30D1**

I. Immunisation des souris

Quatre souris Balb/c femelles âgées de 6 semaines ont été injectées en sous-cutané (SC) dans les coussinets plantaires à trois reprises avec 10 μg de l'anticorps humain anti-domaine A2 du FVIII BOIIB2 suspendu dans un adjuvant complet de Freund (ACF) (1ère immunisation) puis incomplet (AIF).
Une première saignée (saignée 0) a été effectuée avant immunisation (saignée à J0), puis les injections et saignées se sont succédées comme suit :

J1: Injection N°1 (10 μg d'anticorps BOIIB2 en présence d'adjuvant complet de Freund)
J15: Saignée N°1
J16: Injection N°2 (10 μg d'anticorps BOIIB2 en présence d'adjuvant incomplet de Freund)
J28: Saignée N°2
J29: Injection N°3 (10 μg d'anticorps BOIIB2 en présence d'adjuvant incomplet de Freund)
J44: Saignée N°3

II. Evaluation de la réponse immune des souris

De manière à évaluer la présence d'anticorps anti-BOIIB2 dans les différentes saignées, un test ELISA en fixation directe est pratiqué. Dans ce but, l'anticorps BOIIB2 à 3 μg/ml, 50 μl/puits, dans du Tampon Glycine, ON (over night) à 4°C (Tampon Glycine = Glycine 0.1M, NaCl 0.17M, pH 9.2) est insolubilisé. Trois lavages avec du PBS/Tween sont effectués (PBS = NaCl 140.0 mM, KCl 2.6 mM, $KH_2PO_4$ 1.4 mM, $Na_2HPO_4$. $2H_2O$ 8.1 nM, pH 7.4). Le système est laissé à saturation pendant 30 minutes à température ambiante (RT) avec 100 μl/puits de Magic Buffer (Magic Buffer = Tris 50mM, Nacl 0.17M, BSA 1%, pH 7.2). Les saignées sont ensuite diluées à 1/10, 1/50, 1/250 et 1/1250 dans le Magic Buffer et incubées pendant 2 heures à température de chambre (RT) (50 μl/puits). Puis 3 lavages sont effectués dans du PBS/Tween. Le système est ensuite incubé avec une solution d'anticorps polyclonaux de chèvre anti-IgG de souris marqués à la HRP (horseradish peroxidase) (Bio-Rad) à 1 μg/ml pendant 2 heures à température ambiante (RT) (50μl/puits) (dilution dans le Magic Buffer). Le système est ensuite lavé 3 fois avec du PBS/Tween, puis on effectue une révélation avec un chromogène (Ortho-phényl diamine) et lecture de l'intensité de la coloration obtenue par un lecteur avec les filtres correspondant aux longueurs d'ondes 490/650 nm (lecteur Emax Molecular Devices, Sunnyvale, CA).

Résultat des densités optiques obtenues :

| Dilution 250X | Souris 1 | Souris 2 | Souris 3 | Souris 4 |
|---|---|---|---|---|
| Saignée 0 | 0.012 | 0.013 | 0.006 | 0.016 |
| saignée 1 | 0.255 | 0.273 | 0.245 | 0.254 |
| Saignée 2 | 0.357 | 0.388 | 0.366 | 0.386 |
| saignée 3 | 0.362 | 0.34 | 0.334 | 0.357 |

Les résultats obtenus sont reportés dans la figure 7. Conclusion : chaque souris a répondu correctement et de façon similaire à l'injection de l'anticorps BOIIB2. De façon arbitraire, la souris N°2 a été sélectionnée pour effectuer la fusion.

III. <u>Fusion et criblage</u>

Les lymphocytes de la rate de la souris n°2 ont été fusionnés avec les cellules du myélome SP2/0. La fusion a été réalisée de manière classique pour l'homme du métier (J.G. Gilles et al., Blood (2004) 103 : 2617-23 ; P. Cornelis, « Les anticorps monoclonaux», Revue IRE, vol. 7, N°4, 1983).
Les cellules ont été expandues successivement en milieu DMEM (Dulbecco's Modified Eagle Medium) contenant de l'hypoxanthine et de la thymidine selon le principe des dilutions limites et les clones testés positifs détectés par un test en fixation directe ELISA tel que décrit précédemment au point II.
La spécificité de la fixation a été confirmée au moyen d'un anticorps IgG4 humain de spécificité non relevante anti-Der P2 (AK6A3, anticorps dirigé contre un des allergènes majeurs de l'acarien Dermatophagoïdes Pteronisinus (DPT)) disponible dans notre laboratoire. Il est évident pour l'homme du métier que tout autre anticorps d'isotype autre qu'IgG4 peut être utilisé comme contrôle négatif.
Afin de déterminer si les hybridomes sont stables, nous avons répété le test (tests 1 à 3) au cours de l'expansion des clones, dans différents volumes de milieu allant de 200 µl à 5 ml.
Test 1 = mesure dans puits de 200 µl
Test 2 = mesure dans puits de 1 ml
Test 3 = mesure dans bouteille de 5 ml
Résultat des densités optiques obtenues :

| clone | Test 1 | | Test 2 | | Test 3 | |
|---|---|---|---|---|---|---|
| | BOIIB2 | IgG4 (AK6A3) | BOIIB2 | IgG4 (AK6A3) | BOIIB2 | IgG4 (AK6A3) |
| 10C11 | 0,353 | 0,094 | clone mort | clone mort | clone mort | clone mort |
| 10H7 0,436 | | 0,093 | clone mort | clone mort | clone mort | clone mort |
| 11F5 | 0,134 | 0,040 | clone mort | clone mort | clone mort | clone mort |
| 12F5 | 0,291 | 0,087 | 0,024 | 0,002 | 0,012 | 0,002 |
| 14E7 | 0,231 | 0,077 | 0,234 | 0,006 | 0,216 | 0,003 |
| 25F7 | 0,362 | 0,071 | 0,205 | 0,003 | 0,178 | 0,002 |
| 27B5 | 0,333 | 0,046 | 0,238 | 0,003 | 0,259 | 0,003 |
| 28C5 | 0,125 | 0,077 | clone mort | clone mort | clone mort | clone mort |
| 30D1 | 0,324 | 0,093 | 0,311 | 0,003 | 0,293 | 0,002 |

Conclusion : 4 clones sont positifs en fixation directs à l'anticorps BOIIB2 : 14E7, 25F7, 27B5, 30D1. Ces clones sont stables dans les trois tests.

IV. Test d'inhibition avec les surnageants de culture

Afin d'effectuer la sélection d'un clone producteur d'anticorps anti-idiotypiques (Ac anti-Id) qui reconnait précisément un déterminant épitopique localisé au niveau du paratope de l'anticorps BOIIB2 parmi les 4 clones retenus au point III, les anticorps anti-Id ont été testés dans un test ELISA d'inhibition de fixation du BOIIB2 au FVIII insolubilisé. Le facteur VIII recombinant (recFVIII) (Baxter) à 2 µg/ml dans un tampon glycine, 50 µl/puits, a été insolubilisé puis laissé pendant 2 heures à température de chambre. L'anticorps BOIIB2 (ou une IgG4 non relevante) à 0,4 μg/ml final a été pré-incubé pendant 2 heures avec les surnageants de culture des anticorps 14E7, 25F7, 27B5, 30D1 à la dilution 1/1, 1/2 et 1/4 dans le Magic Buffer. Un lavage des puits à 3 reprises est effectué avec un tampon PBS/Tween, puis une saturation avec 100 μl/puits de Magic Buffer (30 min à température de chambre) est effectuée. On incube ensuite avec 50 μl BOIIB2 (ou IgG4 non relevante) et le surnageant de culture (2H, RT, Magic Buffer), puis trois lavages sont effectués. Les anticorps BOIIB2 fixés au recFVIII insolubilisé sont détectés par ajout de 50 μl/puits d'une solution à 1 μg/ml dans le Magic Buffer d'anticorps polyclonaux de souris anti-IgG humaines marqués-HRP (Southern Biotechnology). Trois lavages successifs sont effectués avec du PBS/Tween, puis on effectue la révélation avec un chromogène (OPD ortho-phényl diamine) et une lecture de l'intensité de la coloration obtenue par un lecteur avec les filtres correspondant aux longueurs 490/650 nm ( lecteur Emax Molecular Devices, Sunnyvale, CA).

L'inhibition se calcule comme suit :

$$I(\%) = 1-(\text{signal avec anticorps anti-idiotypique/signal sans anticorps anti-idiotypique})$$

Résultats obtenus :

| Clones | Inihibition (%), dilution 1/1 |
|---|---|
| 14E7 | 0 |
| 30D1 | 73 |
| 27B5 | 0 |
| 25F7 | 0 |

Conclusions : seul un clone (30D1) est capable d'inhiber spécifiquement la fixation de l'anticorps BOIIB2 au recFVIII insolubilisé.

V. Production des 4 clones producteurs des anticorps anti-BOIIB2 de façon extensive

Afin de disposer de quantités d'anticorps supérieures permettant de confirmer la spécificité des anticorps 14E7, 25F7, 27B5, 30D1 de l'exemple IV, nous avons produit ces anticorps anti-idiotypiques en milieu de culture DMEM suivi d'une purification sur colonne d'affinité Protéine G qui permet de purifier puis de concentrer les anticorps et ainsi de s'assurer plus avant de la spécificité des anticorps anti-idiotypiques obtenus.
La concentration d'anticorps purifiés a été déterminée par photométrie, de manière classique pour l'homme du métier.
30D1 : production de 4 ml à 3,23 mg/ml
27B5 : production de 7,5 ml à 3,24 mg/ml
14E7 : production de 4 ml à 2,74 mg/ml
25F7 : production de 5 ml à 0,392 mg/ml

Les quantités obtenues des différents anticorps sont alors :

30D1 : 12.92 mg
27B5 : 24.3 mg
14E7 : 10.96 mg
25F7 : 1.96 mg

VI. <u>Evaluation de la spécificité</u>

Les différentes préparations sont évaluées en ELISA suivant le même protocole que celui décrit au point II et IV.

6.1 Test ELISA : fixation directe des anticorps anti-idiotypiques 14E7, 25F7, 27B5, 30D1_aux anticorps BOIIB2 insolubilisés

Les résultats sont donnés dans la figure 6. Les quatre anticorps se lient de façon dose-dépendante à l'anticorps BOIIB2 insolubilisé sur plaque. Les résultats suggèrent une affinité différente des quatre anticorps, qui est décroissante dans l'ordre 30D1, 27B5, 14E7, 25F7.

6.2. Test ELISA : inhibition de la fixation de l'anticorps BOIIB2 au recFVIII insolubilisé

Le protocole suivi est le même que le protocole décrit au point IV.
La concentration de BOIIB2 utilisée est égale à 0.4 μg/ml, qui est la concentration capable d'inhiber environ 90% de l'activité d'une unité de FVIII.
L'inhibition est calculée comme suit :

I(%) = 1-(signal avec anticorps anti-idiotypique/signal sans anticorps anti-idiotypique)

| | Pourcentage d'inhibition | | | |
|---|---|---|---|---|
| Conc. μg/ml | 30D1 | 27B5 | 14E7 | 25F7 |
| 50 | 94.8 | 86.9 | 0 | 0 |
| 16.7 | 94.8 | 62.7 | 0 | 0 |
| 5.56 | 94.8 | 42.8 | 0 | 0 |
| 1.85 | 92.7 | 14.4 | 0 | 0 |
| 0.62 | 90.6 | 17.1 | 0 | 0 |
| 0.21 | 46 | 16.5 | 0 | 0 |
| 0.07 | 28 | 16.7 | 0 | 0 |
| 0.023 | 17.5 | 16.8 | 0 | 0 |
| 0.0076 | 0 | 0 | 0 | 0 |
| 0.0025 | 0 | 0 | 0 | 0 |

Les résultats sont donnés dans la figure 7. Les anticorps 30D1 et 27B5 montrent un effet inhibiteur tandis que les anticorps 14E7 et 25F7 sont négatifs. Les effets inhibiteurs des anticorps 30D1 et 27B5 se distinguent quantitativement. Tandis que le 30D1 provoque une inhibition à de très faibles doses (90% d'inhibition à 0.6 μg/ml et un maximum de 94.8% à moins de 6 μg/ml), il faut utiliser environ 10 μg/ml pour atteindre 50% d'inhibition.

6.3. Test fonctionnel : mesure de la neutralisation de l'activité inhibitrice (anti-FVIII) de l'anticorps BOIIB2

En fonction des résultats obtenus dans l'exemple précédent, seul l'anticorps 30D1 est évalué.
L'anticorps BOIIB2 est incubé à différentes concentrations (de 1.6 à 0.05 μg/ml) avec l'anti-Id 30D1 (courbe de dilution allant de 1.25 à 0.02 μg/ml final) dans du Magic Buffer est effectuée. Après 30 min, le FVIII Kogenate (Bayer) à 0.5 U/ml final est ajouté puis une incubation complémentaire de 30 min à 37°C est effectuée. Les échantillons sont dilués 30X dans le Magic Buffer, puis les réactifs du test Chromogénique DADE (facteur VIII chromogénique, Dade Behring Gmbh, Marburg, Germany) sont ajoutés selon le mode d'emploi du producteur.
Résultats :

| BOIIB2 (μg/ml) | 0.2 μg/ml | 0.1 μg/ml | 0.05 μg/ml |
|---|---|---|---|
| 30D1 (μg/ml) | | | |
| 1.25 | 100 | 100 | 100 |
| 0.63 | 100 | 100 | 100 |
| 0.32 | 100 | 100 | 100 |
| 0.16 | 54.6 | 90.5 | 100 |
| 0.08 | 0.9 | 61.9 | 100 |
| 0.04 | 0 | 17.6 | 62 |

(suite)

| BOIIB2 (μg/ml) | 0.2 μg/ml | 0.1 μg/ml | 0.05 μg/ml |
|---|---|---|---|
| 0.02 | 0 | 3.9 | 37.3 |

Les résultats sont repris dans la figure 6. Les résultats montrent que l'anticorps 30D1 neutralise l'activité inhibitrice du BOIIB2 de façon dose-dépendante.
L'inhibition est calculée comme suit :

I(%) = 1-(signal avec anticorps anti-idiotypique/signal sans anticorps anti-idiotypique)

6.4 Mesure de la cinétique de fixation de l'anticorps anti-Id 30D1 par la méthode « Surface plasmon resonance BIAcore »

La cinétique de fixation de l'anticorps anti-idiotypique 30D1 sur l'anticorps inhibiteur BOIIB2 a été évaluée par la méthode « Surface plasmon resonance Biacore » en utilisant le Pharmacia Biosensor BIAcore (Pharmacia Biosensor AB, Uppsala, Suède). Les anticorps BOIIB2 ont été immobilisés sur une surface activée d'une sonde CM5. Les anticorps anti-idiotypqies 30D1 ont été infusés avec plusieurs concentrations de BOIIB2 immobilisés sur la surface de la sonde. Les constantes d'association et de dissociation ont été déterminées :

$K_a$ (M-1S-1) = $6 \cdot 10^4$
$K_d$ (S-1) = $1 \cdot 10^{-5}$
$K_D$ : $1 \cdot 10^{-5}/6 \cdot 10^4 = 0.17 \cdot 10^{-9}$ M.

6.5 Caractérisation de la sous-classe de l'anticorps 30D1 Afin de déterminer la sous-casse de l'ac 30D1, le système IsoStrip de Roche a été utilisé (languette colorimétrique). L'anticorps 30D1 est une IgG1, Kappa.

6.6 Séquence

Pour le séquençage, l'ARNm des hybridomes produisant l'anticorps anti-idiotypique 30D1 a été isolée en utilisant le Quick Prep Micro mRNA Purification Kit (Amersham Pharmacia Biotech, Uppsala, Suède). L'ADNc a été synthétisé au moyen du First-strand cDNA Synthesis Kit (Amersham Pharmacia Biotech). L'ADNc codant pour la chaîne lourde (VH) et pour la chaîne légère (VL) a été amplifié par PCR (Polymerase Chain Reaction) au moyen d'amorces spécifiques correspondant aux différentes familles de gènes potentiellement rencontrées chez la souris. Les produis de la PCR ont été isolés d'un gel d'agarose 1.5% au moyen du QIA quick Gel Extraction Kit (Qiagen, Hilden, Germany) et clonés au moyen du pGEM-T Easy Vector system (promega, Madison, WI). L'ADN plasmidique des colonies positives a été isolé au moyen de High Pure Plasmid Isolation Kit (Roche Diagnostics, Mannheim, Germany) et séquencé dans les deux sens avec la Seqenase (US Biochemical, Cleveland, OH).

**Exemple 5 : Neutralisation de l'effet inhibiteur du mélange de l'anticorps BO2C11 et de l'anticorps BOIIB2 par les anticorps anti-idiotypiques 14C12 et 30D1.**

Un essai Bethesda a été effectué au moyen des anticorps monoclonaux B02C11 et BOIIB2 afin d'évaluer la capacité des anticorps anti-idiotypiques 30D1 et 14C12 (anticorps dirigé contre un anticorps inhibiteur se liant au domaine C2 du facteur VIII, décrit dans le document brevet WO 2004/014955) à neutraliser la capacité d'inhibition des anticorps humains anti-FVIII. La concentration en anticorps B02C11 (0,03μg/ml) + BOIIB2 (0,013μg/ml) est définie comme la concentration inhibant 80% de l'activité du FVIII dans le test Bethesda.
L'effet de la neutralisation est évalué :

1° pour chaque anticorps anti-idiotypique ajouté séparément (14C12 --♦-- et 30D1 --■--) à concentration croissante (de 0,0063 à 6,4 μg/ml) au mélange BO2C11 + BOIIB2.
2° pour l'association des 2 anticorps anti-idiotypique . 14C12 et 30D1 (--▲--) ajoutés aux mêmes concentrations au mélange BO2C11 + BOIIB2.

La figure 7 montre que l'utilisation d'une combinaison de 2 anticorps anti-idiotypiques ciblant des anticorps inhibiteurs dirigés contre des domaines différents du FVIII (domaines A2 et C2) possède un effet supérieur à l'utilisation d'un seul anticorps anti-idiotypique.

## Revendications

1. Anticorps monoclonal anti-idiotypique dirigé contre un anticorps inhibiteur du facteur VIII dirigé contre le domaine A2 du facteur VIIII et capable de neutraliser l'activité inhibitrice de l'anticorps inhibiteur cible, **caractérisé en ce que** la région variable de chacune de ses chaînes légères est codée par la séquence d'acide nucléique SEQ ID NO : 2, et **en ce que** la région variable de chacune des chaînes lourdes est codée par la séquence d'acide nucléique SEQ ID NO : 1.

2. Anticorps monoclonal anti-idiotypique dirigé contre un anticorps inhibiteur du facteur VIII dirigé contre le domaine A2 du facteur VIIII et capable de neutraliser l'activité inhibitrice de l'anticorps cible, **caractérisé en ce que** la séquence peptidique de la région variable de chacune de ses chaînes légères est une séquence possédant au moins 70% d'identité avec la séquence SEQ ID NO : 4, et séquence peptidique de la région variable de chacune des chaînes lourdes est une séquence possédant au moins 70% d'identité avec la séquence SEQ ID NO : 3.

3. Anticorps selon la revendication 2, **caractérisé en ce que** la séquence peptidique déduite de la séquence SEQ ID NO : 2 est la séquence SEQ ID NO : 4 et **en ce que** la séquence peptidique déduite de la séquence SEQ ID NO : 1 est la séquence SEQ ID NO : 3.

4. Anticorps selon l'une quelconque des revendications 1 à 3, **caractérisé en ce que** ledit anticorps anti-idiotypique est un anticorps murin.

5. Anticorps selon la revendication 4, **caractérisé en ce que** ledit anticorps anti-idiotypique est une IgG1 kappa.

6. Anticorps selon l'une quelconque des revendications 1 à 3, **caractérisé en ce que** ledit anticorps anti-idiotypique est un anticorps chimérique.

7. Anticorps selon la revendication 6, **caractérisé en ce que** ledit anticorps anti-idiotypique est un anticorps hybride humain.

8. Anticorps selon l'une quelconque des revendications 1 à 3, **caractérisé en ce que** ledit anticorps anti-idiotypique est un anticorps humanisé.

9. Anticorps selon l'une quelconque des revendications 1 à 8, **caractérisé en ce qu'**il s'agit d'un fragment F(ab')2, d'un fragment Fab', ou d'un fragment Fab.

10. Anticorps selon l'une quelconque des revendications 1 à 5, **caractérisé en ce qu'**il est susceptible d'être produit par l'hybridome 30D1 déposé sous le numéro d'enregistrement CNCM 1-3450 à la Collection Nationale de Culture de Microorganismes (CNCM).

11. Lignée cellulaire stable produisant un anticorps monoclonal anti-idiotypique selon l'une quelconque des revendications 1 à 9.

12. Lignée cellulaire stable selon la revendication 11 choisie parmi le groupe consistant en : SP2/0, YB2/0, IR983F, le myélome humain Namalwa, PERC6, les lignées CHO, notamment CHO-K-1, CHO-Lec10, CHO-Lec1, CHO-Lec13, CHO Pro-5, CHO dhfr-, Wil-2, Jurkat, Vero, Molt-4, COS-7, 293-HEK, BHK, K6H6, NSO, SP2/0-Ag 14 et P3X63Ag8.653.

13. Hybridome 30D1 déposé sous le numéro d'enregistrement CNCM 1-3450 à la Collection Nationale de Culture de Microorganismes (CNCM).

14. Fragment d'ADN de séquence SEQ ID NO : 1 codant pour la région variable de la chaine lourde d'un anticorps monoclonal anti-idiotypique selon l'une quelconque des revendications 1 à 10.

15. Fragment d'ADN de séquence SEQ ID NO : 2 codant pour la région variable de la chaine légère d'un anticorps monoclonal anti-idiotypique selon l'une des revendications 1 à 10.

16. Composition pharmaceutique comprenant un anticorps monoclonal anti-idiotypique selon quelconque des revendications 1 à 10 et au moins un excipient et/ou au moins un véhicule pharmaceutiquement acceptables.

**17.** Composition selon la revendication 16, **caractérisée en ce qu'**elle comprend au moins un anticorps anti-idiotypique dirigé contre un anticorps inhibiteur dirigé contre un domaine du facteur VIII autre que le domaine A2.

**18.** Composition selon l'une quelconque des revendications 16 ou 17, **caractérisée en ce qu'**elle comprend un anticorps anti-idiotypique dirigé contre le domaine C2 du facteur VIII.

**19.** Utilisation d'un anticorps monoclonal anti-idiotypique selon l'une quelconque des revendications 1 à 10, pour la fabrication d'un médicament.

**20.** Utilisation d'un anticorps selon l'une quelconque des revendications 1 à 10, pour la fabrication d'un médicament destiné au traitement de l'hémophilie de type A, ladite hémophilie de type A étant une hémophilie avec inhibiteurs.

**21.** Utilisation d'un anticorps selon l'une quelconque des revendications 1 à 10, pour neutraliser in vitro l'activité inhibitrice d'un anticorps inhibiteur dirigé contre le domaine A2 du facteur VIII.

**22.** Utilisation d'un anticorps selon l'une quelconque des revendications 1 à 10, pour la détection in vitro et/ou la purification d'anticorps inhibiteurs du facteur VIII.

## Claims

**1.** An anti-idiotypic monoclonal antibody directed against a factor VIII inhibitory antibody directed against the domain A2 of the factor VIIII and capable of neutralizing the inhibitory activity of the target inhibitory antibody, **characterized in that** the variable region of each of its light chains is coded by the nucleic acid sequence SEQ ID NO: 2, and **in that** the variable region of each of the heavy chains is coded by the nucleic acid sequence SEQ ID NO: 1.

**2.** An anti-idiotypic monoclonal antibody directed against a factor VIII inhibitory antibody directed against the domain A2 of the factor VIIII and capable of neutralizing the inhibitory activity of the target antibody, **characterized in that** the peptide sequence of the variable region of each of its light chains is a sequence having at least 70% identity with the sequence SEQ ID NO: 4, and the peptide sequence of the variable region of each of the heavy chains is a sequence having at least 70% identity with the sequence SEQ ID NO: 3.

**3.** The antibody according to claim 2, **characterized in that** the peptide sequence inferred from the sequence SEQ ID NO: 2 is the sequence SEQ ID NO: 4 and **in that** the peptide sequence inferred from the sequence SEQ ID NO: 1 is the sequence SEQ ID NO: 3.

**4.** The antibody according to any of claims 1 to 3, **characterized in that** said anti-idiotypic antibody is a murine antibody.

**5.** The antibody according to claim 4, **characterized in that** said anti-idiotypic antibody is an IgG1 kappa antibody.

**6.** The antibody according to any of claims 1 to 3, **characterized in that** said anti-idiotypic antibody is a chimeric antibody.

**7.** The antibody according to claim 6, **characterized in that** said anti-idiotypic antibody is a human hybrid antibody.

**8.** The antibody according to any of claims 1 to 3, **characterized in that** said anti-idiotypic antibody is a humanized antibody.

**9.** The antibody according to any of claims 1 to 8, **characterized in that** it is a fragment F(ab')2, a fragment Fab', or a fragment Fab.

**10.** The antibody according to any of claims 1 to 5, **characterized in that** it may be produced by the hybridoma 30D1 deposited under the registration number CNCM 1-3450 at the Collection Nationale de Culture de Microorganismes (National Microorganism Culture Collection)(CNCM).

**11.** A stable cell line producing an anti-idiotypic monoclonal antibody according to any of claims 1 to 9.

**12.** The stable cell line according to claim 11 selected from the group consisting of: SP2/0, YB2/0, IR983F, the Namalwa human myeloma, PERC6, CHO lines, notably CHO-K-1, CHO-Lec10 CHO-Lec1, CHO-Lec13 CHO-Pro-5, CHO dhfr-, Wil-2, Jurkat, Vero, Molt-4, COS-7, 293-HEK, BHK, K6H6, NSO, SP2/0-Ag 14 and P3X63Ag8.653.

**13.** The hybridoma 30D1 deposited under the registration number CNCM I-3450 at the Collection Nationale de Culture de Microorganismes (CNCM).

**14.** The DNA fragment of sequence SEQ ID NO: 1 coding for the variable region of the heavy chain of one anti-idiotypic monoclonal antibody according to any of claims 1 to 10.

**15.** The DNA fragment of sequence SEQ ID NO: 2 coding for the variable region of the light chain of one anti-idiotypic monoclonal antibody according to one of claims 1 to 10.

**16.** A pharmaceutical composition comprising an anti-idiotypic monoclonal antibody according to any of claims 1 to 10, and at least one excipient and/or at least one pharmaceutically acceptable carrier.

**17.** The composition according to claim 16, **characterized in that** it comprises at least one anti-idiotypic antibody directed against an inhibitory antibody directed against a domain of the factor VIII other than the domain A2.

**18.** The composition according to any of claims 16 or 17, **characterized in that** it comprises an anti-idiotypic antibody directed against the domain C2 of the factor VIII.

**19.** The use of an anti-idiotypic monoclonal antibody according to any of claims 1 to 10, for making a drug.

**20.** The use of an antibody according to any of claims 1 to 10, for making a drug, intended for treating hemophilia of type A, said hemophilia of type A being a hemophilia with inhibitors.

**21.** The use of an antibody according to any of claims 1 to 10 for neutralizing *in vitro* the inhibitory activity of an inhibitory antibody directed against the domain A2 of the factor VIII.

**22.** The use of an antibody according to any of claims 1 to 10, for detecting in vitro and/or purifying inhibitory antibodies of the factor VIII.

## Patentansprüche

**1.** Monoklonaler anti-idiotypischer Antikörper, der gegen einen gegen die Domäne A2 des Faktors VIII gerichteten den Faktor VIII hemmenden Antikörper gerichtet ist und in der Lage ist, die hemmende Wirkung des hemmenden Zielantikörpers zu neutralisieren, **dadurch gekennzeichnet, dass** die variable Region jeder seiner leichten Ketten durch die Nukleinsäuresequenz SEQ ID N0:2 kodiert wird und **dadurch**, dass die variable Region jeder seiner schweren Ketten durch die Nukleinsäuresequenz SEQ ID NO:1 kodiert wird.

**2.** Monoklonaler anti-idiotypischer Antikörper, der gegen einen gegen die Domäne A2 des Faktors VIII gerichteten den Faktor VIII hemmenden Antikörper gerichtet ist und in der Lage ist, die hemmende Wirkung des hemmenden Zielantikörpers zu neutralisieren, **dadurch gekennzeichnet, dass** die Peptidsequenz der variablen Region jeder seiner leichten Ketten eine Sequenz ist, die mindestens 70 % Identität mit der Sequenz SEQ ID NO:4 aufweist und die Peptidsequenz der variablen Region jeder seiner schweren Ketten eine Sequenz ist, die mindestens 70 % Identität mit der Sequenz SEQ ID NO:3 aufweist.

**3.** Antikörper nach Anspruch 2, **dadurch gekennzeichnet, dass** die von der Sequenz SEQ ID NO:2 abgeleitete Peptidsequenz die Sequenz SEQ ID NO:4 ist und **dadurch**, dass die von der Sequenz SEQ ID NO:1 abgeleitete Peptidsequenz die Sequenz SEQ ID NO:3 ist.

**4.** Antikörper nach einem der Ansprüche 1 bis 3, **dadurch gekennzeichnet, dass** der anti-idiotypische Antikörper ein muriner Antikörper ist.

**5.** Antikörper nach Anspruch 4, **dadurch gekennzeichnet, dass** der anti-idiotypische Antikörper ein IgGlkappa ist.

**6.** Antikörper nach einem der Ansprüche 1 bis 3, **dadurch gekennzeichnet, dass** der anti-idiotypische Antikörper ein chimärer Antikörper ist.

7. Antikörper nach Anspruch 6, **dadurch gekennzeichnet, dass** der anti-idiotypische Antikörper ein menschlicher Hybridantikörper ist.

8. Antikörper nach einem der Ansprüche 1 bis 3, **dadurch gekennzeichnet, dass** der anti-idiotypische Antikörper ein humanisierter Antikörper ist.

9. Antikörper nach einem der Ansprüche 1 bis 8, **dadurch gekennzeichnet, dass** es sich um ein F(ab')2 Fragment, ein Fab' Fragment oder ein Fab Fragment handelt.

10. Antikörper nach einem der Ansprüche 1 bis 5, **dadurch gekennzeichnet, dass** er durch die Hybridomazelle 30D1 hergestellt werden kann, die unter der Registriernummer CNCM 1-3450 bei der Collection Nationale de Culture de Microorganismes (CNCM) hinterlegt ist.

11. Stabile Zelllinie, die einen monoklonalen anti-idiotypischen Antikörper nach einem der Ansprüche 1 bis 9 herstellt.

12. Stabile Zelllinie nach Anspruch 11, ausgewählt aus der Gruppe bestehend aus: SP2/0, YB2/0, IR983F, dem menschlichen Namalwa Myelom, PERC6, den CHO Linien, insbesondere CHO-K-1, CHO-Lec10, CHO-Lec1 CHO-Lec13, CHO-Pro-5, CHO dhfr-, Wil-2, Jurkat, Vero, Molt-4, COS-7, 293-HEK, BHK, K6H6, NSO, SP2/0-Ag 14 und P3X63Ag8.653.

13. Hybridomazelle 30D1, die unter der Registriernummer CNCM I-3450 bei der Collection Nationale de Culture de Microorganismes (CNCM) hinterlegt ist.

14. DNA Fragment mit der Sequenz SEQ ID N0:1, das für die variable Region der schweren Kette eines monoklonalen anti-idiotypischen Antikörpers nach einem der Ansprüche 1 bis 10 kodiert.

15. DNA Fragment mit der Sequenz SEQ ID NO:2, das für die variable Region der leichten Kette eines monoklonalen anti-idiotypischen Antikörpers nach einem der Ansprüche 1 bis 10 kodiert.

16. Arzneimittelzusammensetzung umfassend einen monoklonalen anti-idiotypischen Antikörper nach einem der Ansprüche 1 bis 10 und mindestens einen Hilfsstoff und/oder mindestens einen pharmazeutisch verträglichen Trägerstoff.

17. Zusammensetzung nach Anspruch 16, **dadurch gekennzeichnet, dass** sie mindestens einen anti-idiotypischen Antikörper umfasst, der gegen einen hemmenden Antikörper gerichtet ist, der gegen eine andere Domäne des Faktors VIII als die Domäne A2 gerichtet ist.

18. Zusammensetzung nach einem der Ansprüche 16 und 17, **dadurch gekennzeichnet, dass** sie einen anti-idiotypischen Antikörper umfasst, der gegen die Domäne C2 des Faktors VIII gerichtet ist.

19. Verwendung eines monoklonalen anti-idiotypischen Antikörpers nach einem der Ansprüche 1 bis 10 zur Herstellung eines Medikaments.

20. Verwendung eines Antikörpers nach einem der Ansprüche 1 bis 10 zur Herstellung eines Medikaments zur Behandlung von Hämophilie des Typs A, wobei die Hämophilie des Typs A eine Hämophilie mit Hemmstoffen ist.

21. Verwendung eines Antikörpers nach einem der Ansprüche 1 bis 10 zur in Vitro Neutralisierung der hemmenden Aktivität eines hemmenden Antikörpers, der gegen die Domäne A2 des Faktors VIII gerichtet ist.

22. Verwendung eines Antikörpers nach einem der Ansprüche 1 bis 10 zur in vitro Bestimmung und/oder Aufreinigung eines hemmenden Antikörpers des Faktors VIII.

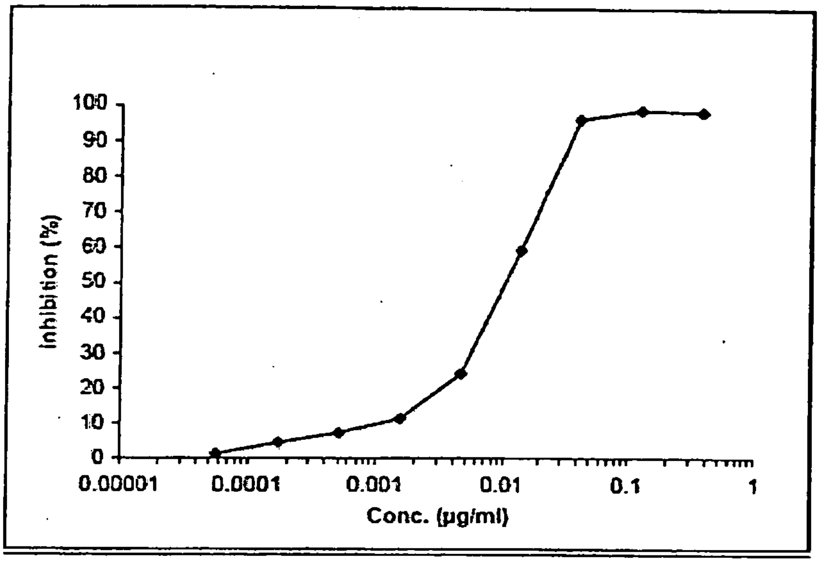
100
90
80
70
60
50
40
30
20
10
0
Inhibition (%)
0.00001
0.0001
0.001
0.01
0.1
1
Conc. (µg/ml)

Fig. 1

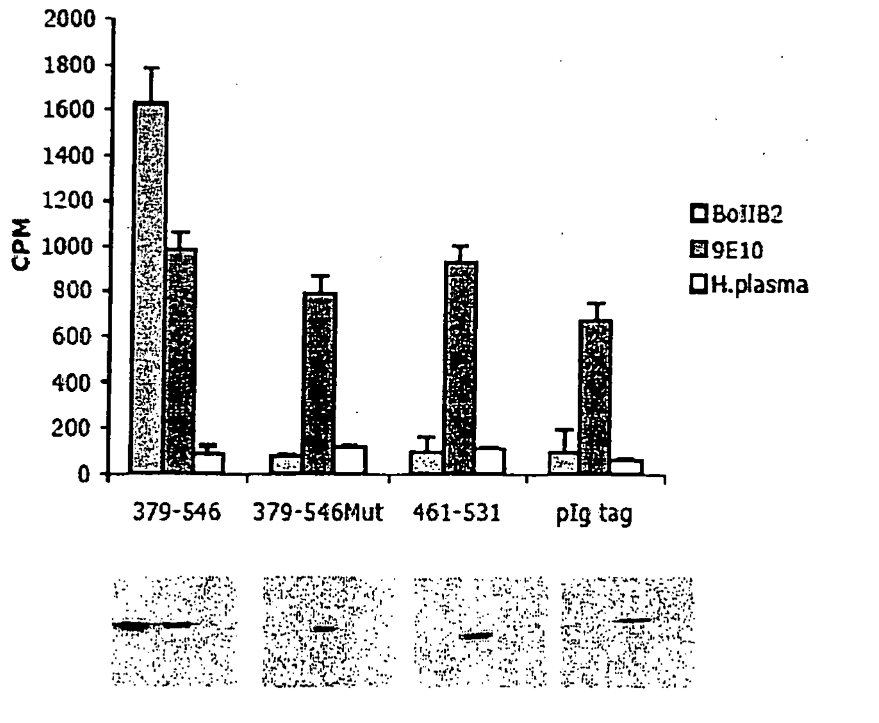
2000
1800
1600
1400
1200
1000
800
600
400
200
0
CPM
BoIIB2
9E10
H.plasma
379-546
379-546Mut
461-531
pIg tag

Fig. 2

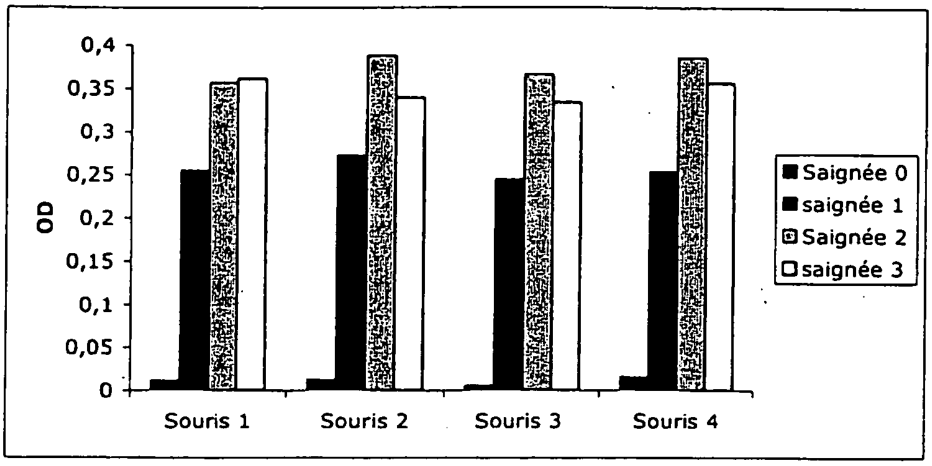
0,4
0,35
0,3
0,25
0,2
0,15
0,1
0,05
0
OD
Saignée 0
saignée 1
Saignée 2
saignée 3
Souris 1
Souris 2
Souris 3
Souris 4

Fig. 3

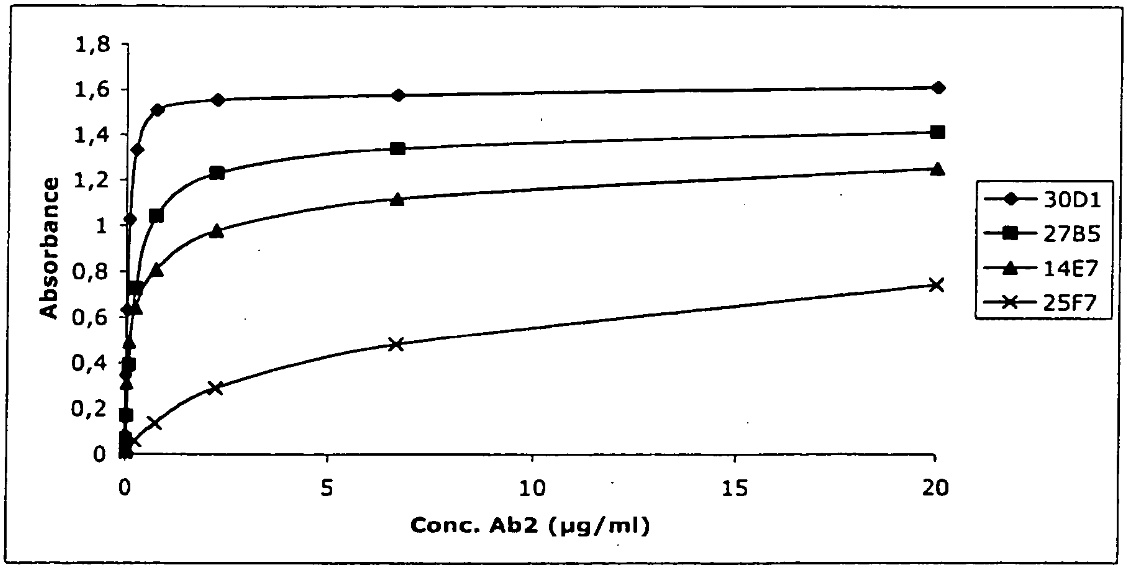
1,8
1,6
1,4
1,2
1
0,8
0,6
0,4
0,2
0
Absorbance
0
5
10
15
20
Conc. Ab2 (µg/ml)
30D1
27B5
14E7
25F7

Fig. 4

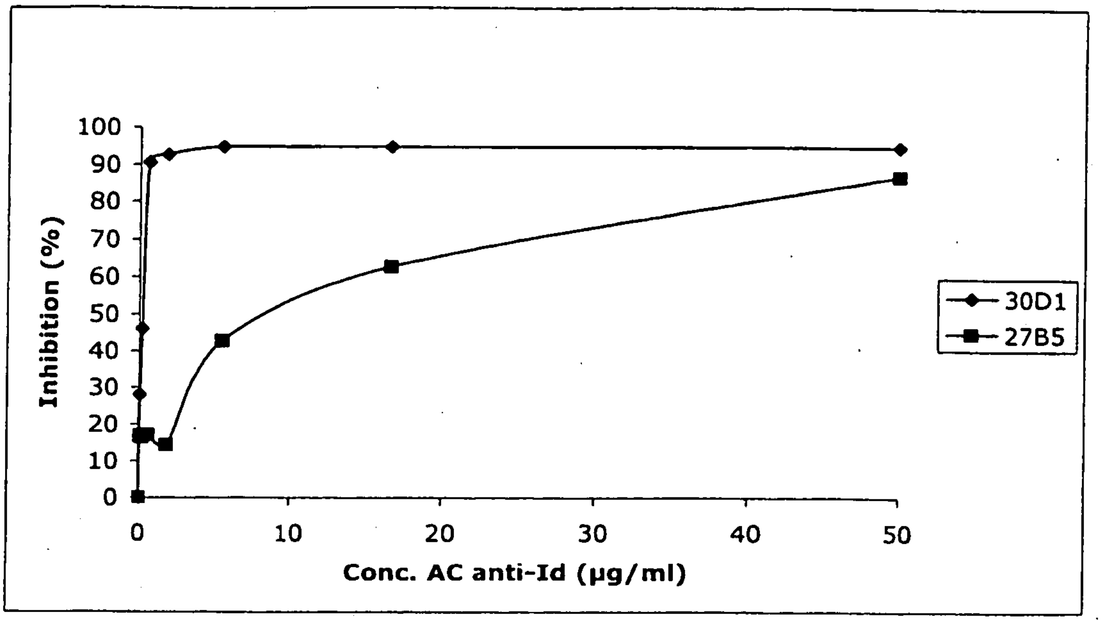
100
90
80
70
60
50
40
30
20
10
0
Inhibition (%)
0
10
20
30
40
50
Conc. AC anti-Id (µg/ml)
30D1
27B5

Fig. 5

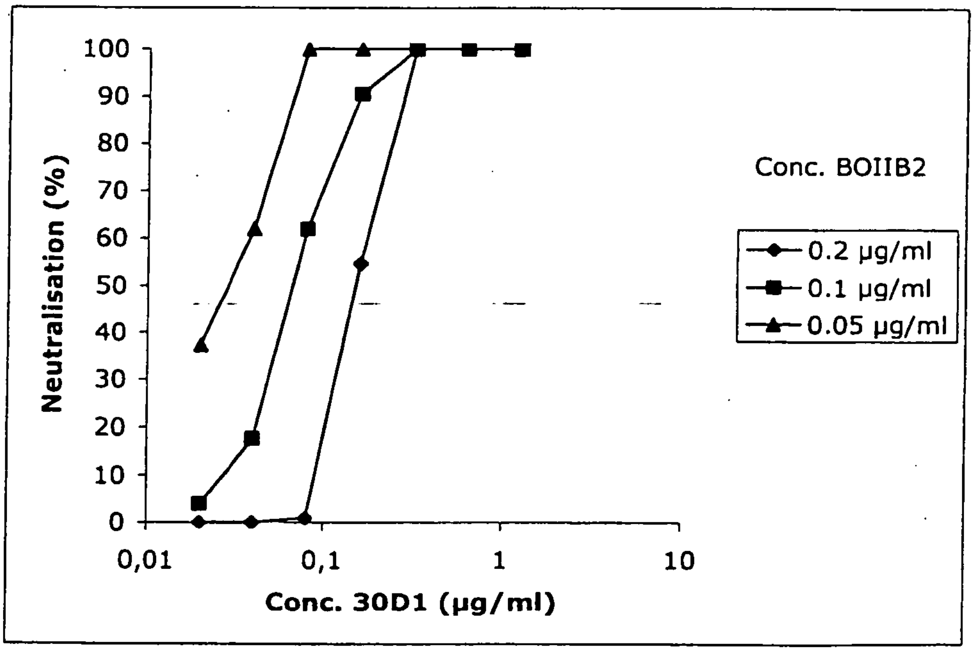
100
90
80
70
60
50
40
30
20
10
0
Neutralisation (%)
0,01
0,1
1
10
Conc. 30D1 (µg/ml)
Conc. BOIIB2
0.2 µg/ml
0.1 µg/ml
0.05 µg/ml

Fig. 6

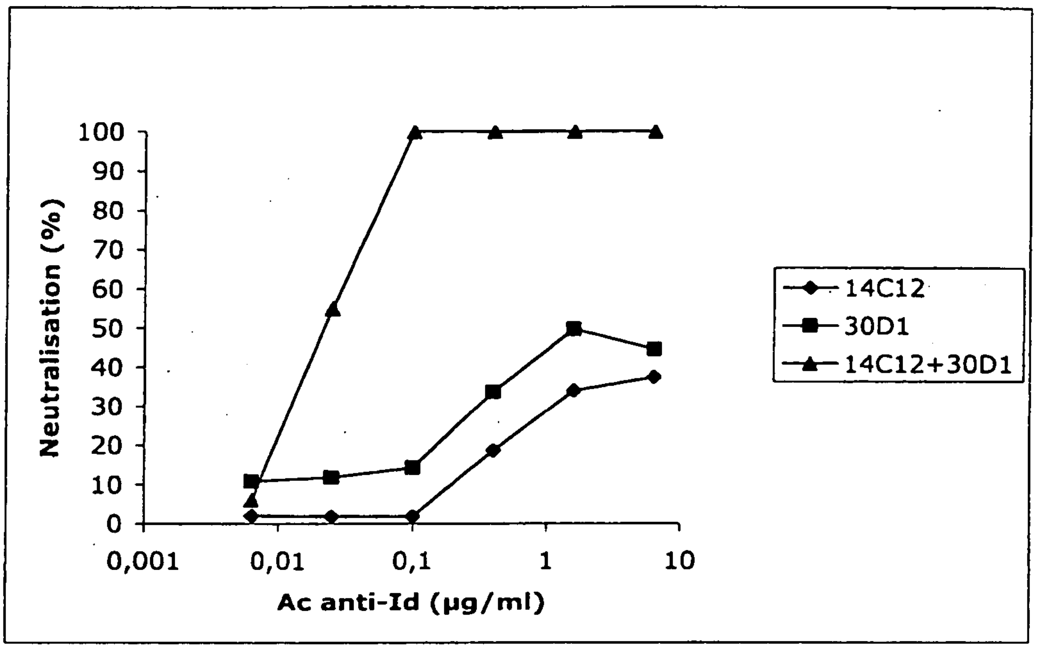
100
90
80
70
60
50
40
30
20
10
0
Neutralisation (%)
0,001
0,01
0,1
1
10
Ac anti-Id (µg/ml)
14C12
30D1
14C12+30D1

Fig. 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2004014955 A


### Littérature non-brevet citée dans la description


- **Wood et al.** *Nature,* 1984, vol. 312, 330-337
- **Gilles JG et al.** *Blood,* 1993, vol. 82, 2452-2461
- **Jarvis et al.** *Thromb Haemost.,* Février 1996, vol. 75 (2), 318-25
- **Jacquemin et al.** *Blood,* 2000, vol. 95, 156-163
- **Jacquemin et al.** *Blood,* 15 Juillet 1998, vol. 92 (2), 496-506
- **Ananyeva NM et al.** *Blood Coagul Fibrinolysis.,* Mars 2004, vol. 15 (2), 109-24
- **Saint-Rémy JM et al.** *Vox Sang,* 1999, vol. 77 (1), 21-24
- **Lollar et al.** *J Clin Invest.,* Juin 1994, vol. 93 (6), 2497-504
- **Fay et al.** *J Biol Chem.,* 1996, vol. 271 (11), 6027-6032
- **Lubahn et al.** *PNAS,* vol. 87 (21), 8232-3236
- **Jacquemin et al.** *Blood,* 1998, vol. 92, 494-506
- **Morrison et al.** *Proc. Natl. Acad. Sci. U.S.A.,* 1984, vol. 81, 6851-55
- **Jones et al.** *Nature,* 1986, vol. 321, 522
- **Riechmann et al.** *Nature,* 1988, vol. 332, 323
- **Madec et al.** *J Immunol,* 1996, vol. 156, 3541-3549
- Current Protocols in Immunology. John Wiley & Sons, Inc, 1994
- Current Protocols in Immunology. Wiley & Sons, Inc, 1994
- **Sato K et al.** *Cancer Research,* 1993, vol. 53, 851-856
- **Jacquemin et al.** *Blood.,* 01 Janvier 2000, vol. 95 (1), 156-63
- **Tomlinson et al.** *MRC Centre for protein Engineering*
- **J.G. Gilles et al.** *Blood,* 2004, vol. 103, 2617-23
- **P. Cornelis.** Les anticorps monoclonaux. *Revue IRE,* 1983, vol. 7 (4

SEQUENCE LISTING

<110> Laboratoire Français du Fractionnement et des Biotechnologies

<120> Anticorps anti-idiotypique neutralisant l'activité inhibitrice d'un anticorps inhibiteur du facteur VIII

<130> anti-A2

<160> 8

<170> PatentIn version 3.1

<210> 1
<211> 461
<212> DNA
<213> Mus musculus

<400> 1

```
atggaatgga gctggatcat tctcttcctc ctgtcaggaa ctgcaggtgt ccactctgag     60
gtccagctgc aacagtctgg acctgagctg gtgaagcctg gagcttcaat gaagatatcc    120
tgcaaggctt ctggttactc attcactgac tacaccatga actgggtgaa gcagagccat    180
ggaaagaacc ttgagtggat tggacttatt aatccttaca atgttatttc ttcctacaac    240
cagaagttca agggcaaggc cacattaact gtagacaagt catccagcac agcctacatg    300
gagctcctca gtctgacatc tgaggactct gcagtctatt actgtgcaag aaagggctac    360
ggtagtagca tgtttgctta ctggggccaa gggactctgg tcactgtctc tgcagccaaa    420
acgacacccc catctgtcta tccactggcc cctgggtctg a                        461
```

<210> 2
<211> 431
<212> DNA
<213> Mus musculus

<400> 2

```
atggagtcac atactcaggt cttcgtattc gtgtttctct ggttgtctgg tgttgacgga     60
gacattgtga tgacccagtc tcacaaattc atgtccacat cagtaggagg cagggtcagc    120
```

atcacctgca aggccagtca ggatgtaact actgctgtag cctggtatca acagaaacca 180

ggacaatctc ctaaactact gatttactcg gcatcctacc ggtacactgg agtccctgat 240

cgcttcactg gcagtggatc tgggacggat ttcactttca ccttcagcag tgtgcaggct 300

gaagacctgg cagtttatta ctgtcagcaa cattatagta ttccgtggac gttcggtgga 360

ggcaccaagc tggaaatcaa acgggctgat gctgcaccaa ctgtatccat cttcccacca 420

tccagtgcgc a 431

<210> 3
<211> 153
<212> PRT
<213> Mus musculus

<400> 3

Met Glu Trp Ser Trp Ile Ile Leu Phe Leu Leu Ser Gly Thr Ala Gly
1 5 10 15

Val His Ser Glu Val Gln Leu Gln Gln Ser Gly Pro Glu Leu Val Lys
20 25 30

Pro Gly Ala Ser Met Lys Ile Ser Cys Lys Ala Ser Gly Tyr Ser Phe
35 40 45

Thr Asp Tyr Thr Met Asn Trp Val Lys Gln Ser His Gly Lys Asn Leu
50 55 60

Glu Trp Ile Gly Leu Ile Asn Pro Tyr Asn Val Ile Ser Ser Tyr Asn
65 70 75 80

Gln Lys Phe Lys Gly Lys Ala Thr Leu Thr Val Asp Lys Ser Ser Ser
85 90 95

Thr Ala Tyr Met Glu Leu Leu Ser Leu Thr Ser Glu Asp Ser Ala Val
100 105 110

Tyr Tyr Cys Ala Arg Lys Gly Tyr Gly Ser Ser Met Phe Ala Tyr Trp
115 120 125

Gly Gln Gly Thr Leu Val Thr Val Ser Ala Ala Lys Thr Thr Pro Pro
130 135 140

Ser Val Tyr Pro Leu Ala Pro Gly Ser
145 150

<210> 4
<211> 143
<212> PRT
<213> Mus musculus

<400> 4

Met Glu Ser His Thr Gln Val Phe Val Phe Val Phe Leu Trp Leu Ser
1               5                   10                  15

Gly Val Asp Gly Asp Ile Val Met Thr Gln Ser His Lys Phe Met Ser
            20                  25                  30

Thr Ser Val Gly Gly Arg Val Ser Ile Thr Cys Lys Ala Ser Gln Asp
        35                  40                  45

Val Thr Thr Ala Val Ala Trp Tyr Gln Gln Lys Pro Gly Gln Ser Pro
    50                  55                  60

Lys Leu Leu Ile Tyr Ser Ala Ser Tyr Arg Tyr Thr Gly Val Pro Asp
65                  70                  75                  80

Arg Phe Thr Gly Ser Gly Ser Gly Thr Asp Phe Thr Phe Thr Phe Ser
                85                  90                  95

Ser Val Gln Ala Glu Asp Leu Ala Val Tyr Tyr Cys Gln Gln His Tyr
            100                 105                 110

Ser Ile Pro Trp Thr Phe Gly Gly Gly Thr Lys Leu Glu Ile Lys Arg
        115                 120                 125

Ala Asp Ala Ala Pro Thr Val Ser Ile Phe Pro Pro Ser Ser Ala
    130                 135                 140

<210> 5
<211> 1392
<212> DNA
<213> Homo sapiens

<400> 5

atgaaacacc tgtggttctt ccttctcctg gtggcagctc ccagatgtgt cctgtcccag 60
gtgcagctgc aggagtcggg cccaggactg gtgaagcctt cggagaccct gtccctcacc 120
tgcactgtct ctggtgactc catcagtgat tactactgga gctggatccg gcagccccca 180
gggaagggac tggagtggat tggctatttt ttttacagtg ggggcagcaa ttacaacccc 240
tccctcaaga gtcgagtcac catgtcagta gacacatcca agaaccagtt ctccctgaag 300
ctgggctctg tgaccgctgc ggacacggcc gtctattact gtgcgagatc gcagttacga 360

```
tattacctgg acgtctgggg ccaagggacc acggtcaccg tctcctcggc ctccaccaag    420
ggcccatcgg tcttcccccct ggcgccctgc tccaggagca cctccgagag cacagcggcc    480
ctgggctgcc tggtcaagga ctacttcccc gaaccggtga cggtgtcgtg gaactcaggc    540
gccctgacca gcggcgtgca caccttcccg gctgtcctac agtcctcagg actctactcc    600
ctcagcagcg tggtgaccgt gccctccagc agcttgggca cgaagaccta cacctgcaat    660
gtagatcaca agcccagcaa caccaaggtg gacaagagag ttgagtccaa atatggtccc    720
ccatgcccat catgcccagc acctgagttc ctggggggac catcagtctt cctgttcccc    780
ccaaaaccca aggacactct catgatctcc cggacccctg aggtcacgtg cgtggtggtg    840
gacgtgagcc aggaagaccc cgaggtccag ttcaactggt acgtggatgg cgtggaggtg    900
cataatgcca agacaaagcc gcgggaggag cagttcaaca gcacgtaccg tgtggtcagc    960
gtcctcaccg tcctgcacca ggactggctg aacggcaagg agtacaagtg caaggtctcc   1020
aacaaaggcc tcccgtcctc catcgagaaa accatctcca aagccaaagg gcagccccga   1080
gagccacagg tgtacaccct gcccccatcc caggaggaga tgaccaagaa ccaggtcagc   1140
ctgacctgcc tggtcaaagg cttctacccc agcgacatcg ccgtggagtg ggagagcaat   1200
gggcagccgg agaacaacta caagaccacg cctcccgtgc tggactccga cggctccttc   1260
ttcctctaca gcaggctaac cgtggacaag agcaggtggc aggaggggaa tgtcttctca   1320
tgctccgtga tgcatgaggc tctgcacaac cactacacac agaagagcct ctccctgtct   1380
ctgggtaaat ga                                                       1392
```

<210> 6
<211> 699
<212> DNA
<213> Homo sapiens

<400> 6

```
atggaaaccc cagckcagct tctcttcctc ctgctactct ggctcccaga taccaccgga     60
gaaattgtgt tgacgcagtc tccaggcacc ctgtctttgt ctccagggga aagagccacc    120
ctctcctgca gggccagtca gagtgttgac agcaactact tagcctggta ccagcagaaa    180
cctggccagg ctcccagggt cgtcatctat ggtgcatcca acagggccac tggcatccca    240
gacaggttca gtggcagtgg gtctgggaca gagttcactc tcaccatcag cagactggac    300
cctgaagatt ttgcagtgta ttactgtcag cagtatggta gcttcttcgg ccaagggaca    360
cgactggaga ttaaacgaac tgtggctgca ccatctgtct tcatcttccc gccatctgat    420
gagcagttga aatctggaac tgcctctgtt gtgtgcctgc tgaataactt ctatcccaga    480
gaggccaaag tacagtggaa ggtggataac gccctccaat cgggtaactc ccaggagagt    540
gtcacagagc aggacagcaa ggacagcacc tacagcctca gcagcaccct gacgctgagc    600
```

aaagcagact acgagaaaca caaagtctac gcctgcgaag tcacccatca gggcctgagc 660

tcgcccgtca caaagagctt caacagggga gagtgttag 699

<210> 7
<211> 463
<212> PRT
<213> Homo sapiens

<400> 7

```
Met Lys His Leu Trp Phe Phe Leu Leu Leu Val Ala Ala Pro Arg Cys
1               5                   10                  15

Val Leu Ser Gln Val Gln Leu Gln Glu Ser Gly Pro Gly Leu Val Lys
            20                  25                  30

Pro Ser Glu Thr Leu Ser Leu Thr Cys Thr Val Ser Gly Asp Ser Ile
        35                  40                  45

Ser Asp Tyr Tyr Trp Ser Trp Ile Arg Gln Pro Pro Gly Lys Gly Leu
    50                  55                  60

Glu Trp Ile Gly Tyr Phe Phe Tyr Ser Gly Gly Ser Asn Tyr Asn Pro
65                  70                  75                  80

Ser Leu Lys Ser Arg Val Thr Met Ser Val Asp Thr Ser Lys Asn Gln
                85                  90                  95

Phe Ser Leu Lys Leu Gly Ser Val Thr Ala Ala Asp Thr Ala Val Tyr
            100                 105                 110

Tyr Cys Ala Arg Ser Gln Leu Arg Tyr Tyr Leu Asp Val Trp Gly Gln
        115                 120                 125

Gly Thr Thr Val Thr Val Ser Ser Ala Ser Thr Lys Gly Pro Ser Val
    130                 135                 140

Phe Pro Leu Ala Pro Cys Ser Arg Ser Thr Ser Glu Ser Thr Ala Ala
145                 150                 155                 160

Leu Gly Cys Leu Val Lys Asp Tyr Phe Pro Glu Pro Val Thr Val Ser
                165                 170                 175

Trp Asn Ser Gly Ala Leu Thr Ser Gly Val His Thr Phe Pro Ala Val
            180                 185                 190

Leu Gln Ser Ser Gly Leu Tyr Ser Leu Ser Ser Val Val Thr Val Pro
        195                 200                 205
```

Ser Ser Ser Leu Gly Thr Lys Thr Tyr Thr Cys Asn Val Asp His Lys
210 215 220

Pro Ser Asn Thr Lys Val Asp Lys Arg Val Glu Ser Lys Tyr Gly Pro
225 230 235 240

Pro Cys Pro Ser Cys Pro Ala Pro Glu Phe Leu Gly Gly Pro Ser Val
245 250 255

Phe Leu Phe Pro Pro Lys Pro Lys Asp Thr Leu Met Ile Ser Arg Thr
260 265 270

Pro Glu Val Thr Cys Val Val Val Asp Val Ser Gln Glu Asp Pro Glu
275 280 285

Val Gln Phe Asn Trp Tyr Val Asp Gly Val Glu Val His Asn Ala Lys
290 295 300

Thr Lys Pro Arg Glu Glu Gln Phe Asn Ser Thr Tyr Arg Val Val Ser
305 310 315 320

Val Leu Thr Val Leu His Gln Asp Trp Leu Asn Gly Lys Glu Tyr Lys
325 330 335

Cys Lys Val Ser Asn Lys Gly Leu Pro Ser Ser Ile Glu Lys Thr Ile
340 345 350

Ser Lys Ala Lys Gly Gln Pro Arg Glu Pro Gln Val Tyr Thr Leu Pro
355 360 365

Pro Ser Gln Glu Glu Met Thr Lys Asn Gln Val Ser Leu Thr Cys Leu
370 375 380

Val Lys Gly Phe Tyr Pro Ser Asp Ile Ala Val Glu Trp Glu Ser Asn
385 390 395 400

Gly Gln Pro Glu Asn Asn Tyr Lys Thr Thr Pro Pro Val Leu Asp Ser
405 410 415

Asp Gly Ser Phe Phe Leu Tyr Ser Arg Leu Thr Val Asp Lys Ser Arg
420 425 430

Trp Gln Glu Gly Asn Val Phe Ser Cys Ser Val Met His Glu Ala Leu
435 440 445

His Asn His Tyr Thr Gln Lys Ser Leu Ser Leu Ser Leu Gly Lys
450 455 460

<210> 8
<211> 232
<212> PRT

<213> Homo sapiens

<400> 8

Met Glu Thr Pro Ala Gln Leu Leu Phe Leu Leu Leu Leu Trp Leu Pro
1               5                   10                  15

Asp Thr Thr Gly Glu Ile Val Leu Thr Gln Ser Pro Gly Thr Leu Ser
            20                  25                  30

Leu Ser Pro Gly Glu Arg Ala Thr Leu Ser Cys Arg Ala Ser Gln Ser
        35                  40                  45

Val Asp Ser Asn Tyr Leu Ala Trp Tyr Gln Gln Lys Pro Gly Gln Ala
    50                  55                  60

Pro Arg Val Val Ile Tyr Gly Ala Ser Asn Arg Ala Thr Gly Ile Pro
65                  70                  75                  80

Asp Arg Phe Ser Gly Ser Gly Ser Gly Thr Glu Phe Thr Leu Thr Ile
                85                  90                  95

Ser Arg Leu Asp Pro Glu Asp Phe Ala Val Tyr Tyr Cys Gln Gln Tyr
            100                 105                 110

Gly Ser Phe Phe Gly Gln Gly Thr Arg Leu Glu Ile Lys Arg Thr Val
        115                 120                 125

Ala Ala Pro Ser Val Phe Ile Phe Pro Pro Ser Asp Glu Gln Leu Lys
    130                 135                 140

Ser Gly Thr Ala Ser Val Val Cys Leu Leu Asn Asn Phe Tyr Pro Arg
145                 150                 155                 160

Glu Ala Lys Val Gln Trp Lys Val Asp Asn Ala Leu Gln Ser Gly Asn
                165                 170                 175

Ser Gln Glu Ser Val Thr Glu Gln Asp Ser Lys Asp Ser Thr Tyr Ser
            180                 185                 190

Leu Ser Ser Thr Leu Thr Leu Ser Lys Ala Asp Tyr Glu Lys His Lys
        195                 200                 205

Val Tyr Ala Cys Glu Val Thr His Gln Gly Leu Ser Ser Pro Val Thr
    210                 215                 220

Lys Ser Phe Asn Arg Gly Glu Cys
225                 230